# EUROPEAN PATENT SPECIFICATION

(11) **EP 1 517 679 B1**
(45) Date of publication and mention of the grant of the patent: **13.02.2008**
(21) Application number: 03732714.5
(22) Date of filing: 17.06.2003
(51) Int. Cl.: A61K 31/19, A61P 3/06, A61P 3/10, C07C 323/62, C07C 235/20

(54) **ORTHO-SUBSTITUTED BENZOIC ACID DERIVATIVES FOR THE TREATMENT OF INSULIN RESISTANCE**
ORTHO-SUBSTITUIERTE BENZOESÄUREDERIVATE ZUR BEHANDLUNG VON INSULINRESISTENZ
DERIVES DE L'ACIDE BENZOIQUE ORTHO-SUBSTITUES DESTINES AU TRAITEMENT DE L'INSULINORESISTANCE

(30) Priority: 20.06.2002 SE 0201936
(43) Date of publication of application: 30.03.2005
(73) Proprietor: AstraZeneca AB, 151 85 Södertälje (SE)
(72) Inventor: LI, Lanna, S-431 83 Mölndal (SE)
(86) International application number: PCT/GB2003/002591
(87) International publication number: WO 2004/000294

(56) References cited:
- EP-A- 1 184 366
- US-A1- 2002 022 656
- US-B1- 6 258 850

## Description

### Field of the invention

The present invention relates to certain novel benzoic acid derivatives, to processes for preparing such compounds, to their utility in treating clinical conditions associated with insulin resistance, to methods for their therapeutic use and to pharmaceutical compositions containing them

### Background of the invention

The Insulin Resistance Syndrome (IRS) including type 2 diabetes mellitus, which refers to a cluster of manifestations including insulin resistance with accompanying hyperinsulinaemia, possible type 2 diabetes mellitus, arterial hypertension, central (visceral) obesity, dyslipidaemia observed as deranged lipoprotein levels typically characterised by elevated VLDL (very low density lipoproteins), small dense LDL particles and reduced HDL (high density lipoprotein) concentrations and reduced fibrinolysis.

Recent epidemiological research has documented that individuals with insulin resistance run a greatly increased risk of cardiovascular morbidity and mortality, notably suffering from myocardial infarction and stroke. In type 2 diabetes mellitus atherosclerosis related conditions cause up to 80% of all deaths.

In clinical medicine there is awareness of the need to increase the insulin sensitivity in IRS suffering patients and thus to correct the dyslipidaemia which is considered to cause the accelerated progress of atherosclerosis. However, currently this is not a universally well defined disease.

The S-enantiomer of the compound of formula C below 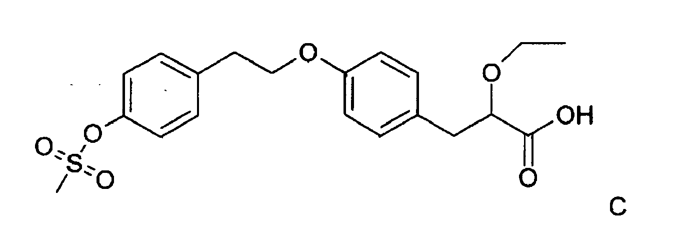 2-ethoxy-3-[4-(2-{4-methanesulfonyloxyphenyl}ethoxy)phenyl]propanoic acid, is disclosed in PCT Publication Number WO99/62872. This compound is reported to be a modulator of peroxisome proliferator-activated receptors (PPAR, for a review of the PPARs see T. M.Willson et al, J Med Chem 2000, Vol 43, 527) and has combined PPARα/PPARγ agonist activity (Structure, 2001, Vol 9, 699, P. Cronet et al). This compound is effective in treating conditions associated with insulin resistance.

EP 1 184 366 A relates to substituted phenylpropanoic acid derivatives, effective for the therapy of abnormality of lipidmetabolism.

US 2002/022656 A1 relates to compounds having two terminal carboxylic acid and/or ester groups that may be used as medicaments.

US 6 258 850 B1 discloses a novel 3-aryl-2-hydroxypropionic acid derivative, which may be used in clinical conditions associated with insulin resistance.

Surprisingly a series of compounds has now been found which are selective PPARα modulators.

### Description of the invention

The present invention provides a compound of formula I 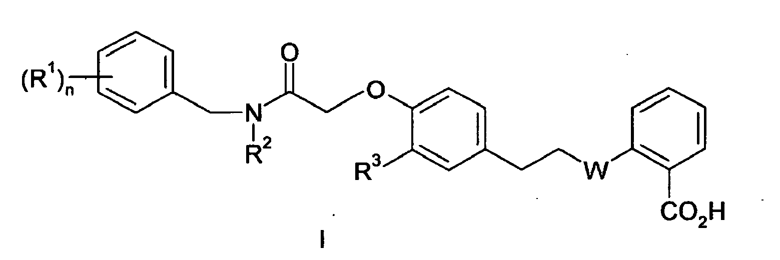 wherein n is 0, 1 or 2;
R¹ represents halo, a C₁₋₄alkyl group which is optionally substituted by one or more fluoro, a C₁₋₄alkoxy group which is optionally substituted by one or more fluoro and wherein when n is 2 the substituents R¹ may be the same or different;
R² represents an unbranched C₂₋₇alkyl group;
R³ represents H or OCH₃; and
W represents O or S
and pharmaceutically acceptable salts thereof.

Further values of R¹, R², R³ and W in compounds of Formula I now follow. It will be understood that such values may be used with any of the definitions, claims or embodiments defined hereinbefore or hereinafter.

In a first aspect R¹ is halo, a C₁₋₄alkyl group or a C₁₋₄alkoxy group and n is 0, 1 or 2. Particularly R¹ is fluoro,chloro or trifluoromethyl when n is 1. Particularly R¹ is fluoro when n is 2.

In a second aspect R² represents ethyl or hexyl.

In a third aspect R³ represents H.

In a fourth aspect R³ represents OMe.

In a fifth aspect W represents O.

In a sixth aspect W represents S.

The term unbranched C₂₋₇alkyl denotes a straight-chain, saturated aliphatic hydrocarbon having from 2 to 7 carbon atoms. Examples of said alkyl include ethyl, n-propyl, n-butyl, n- pentyl, n-hexyl and n- heptyl.

It will be understood by those skilled in the art that the term interrupted as used above means that the oxygen atom is situated within the alkyl chain and is not the terminal atom.

The compounds of formula I have activity as medicaments, in particular the compounds of formula I are selective agonists of PPARα, that is, their EC₅₀ for PPARα is at least four times lower and preferably at least 10 or 50 times lower than their respective EC₅₀ for PPARγ wherein the EC₅₀s are measured and calculated as described in the assays later in this document. The compounds of formula I are potent and selective.

The present invention provides a compound selected from:
2-[2-(4-{2-[ethyl(2-fluorobenzyl)amino]-2-oxoethoxy}phenyl)ethoxy]benzoic acid;
2-[2-(4-{2-[(2,4-difluorobenzyl)(heptyl)amino]-2-oxoethoxy}-3-methoxyphenyl)-ethoxy]benzoic acid;
2-[2-(4-{2-[(4-chlorobenzyl)(ethyl)amino]-2-oxoethoxy}phenyl)ethylthio]benzoic acid;
2-[2-(4-{2-[(4-chlorobenzyl)(ethyl)amino]-2-oxoethoxy}phenyl)ethoxy]benzoic acid;
2-[2-(4-{2-[ethyl(4-trifluoromethylbenzyl)amino]-2-oxoethoxy}phenyl)ethoxy]benzoic acid;
2-[2-(4-{2-[ethyl(4-trifluoromethylbenzyl)amino]-2-oxoethoxy}phenyl)ethylthio]benzoic acid; and
2-{2-[4-(2-{butyl[2-fluoro-4-(trifluoromethyl)benzyl]amino}-2-oxoethoxy)phenyl]-ethoxy}benzoic acid;
2-[2-(4-{2-[(2,4-difluorobenzyl)(propyl)amiuo]-2-oxoethoxy}phenyl)ethoxy]benzoic acid;
2-[2-(4-{2-[benzyl(ethyl)ainino]-2-oxoethoxy}phenyl)ethoxy]benzoic acid;
2-{[2-(4-{2-[benzyl(ethyl)amino]-2-oxoethoxy}phenyl)ethyl]thio}benzoic acid;
2-[2-(4-{2-[(4-*tert*-butylbenzyl)(ethyl)amino]-2-oxoethoxy}phenyl)ethoxy]benzoic acid;
2-[2-(4-{2-[ethyl(4-fluorobenzyl)amino]-2-oxoethoxy}phenyl)ethoxy]benzoic acid;
2-{[2-(4-{2-[ethyl(2-fluorobenzyl)amino]-2-oxoethoxy}phenyl)ethyl]thio}benzoic acid; or 2-{[2-(4-{2-[(2-chlorobenzyl)(ethyl)amino]-2-oxoethoxy}phenyl)ethyl]thio}benzoic acid and pharmaceutically acceptable salts thereof.

Particularly the compound is selected from:
2-[2-(4-(2-[ethyl(2-fluorobenzyl)amino]-2-oxoetlioxy)phenyl)ethoxy]benzoic acid;
2-[2-(4-{2-[(4-chlorobenzyl)(ethyl)amino]-2-oxoethoxy}phenyl)ethylthio]benzoic acid;
2-[2-(4-{2-[(2,4-difluorobenzyl)(propyl)amino]-2-oxoethoxy}phenyl)ethoxy]benzoic acid;
2-[2-(4-{2-[benzyl(ethyl)amino]-2-oxoethoxy}phenyl)ethoxy]benzoic acid; or
2-[2-(4-{2-[ethyl(4-fluorobenzyl)amino]-2-oxoethoxy}phenyl)ethoxy]benzoic acid;

It will also be understood that certain compounds of the present invention may exist in solvated, as well as unsolvated forms. It is to be understood that the present invention encompasses all such solvated forms. Certain compounds of the present invention may exist as tautomers. It is to be understood that the present invention encompasses all such tautomers.

Throughout the specification and the appended claims, a given chemical formula or name shall encompass all stereo and optical isomers and racemates thereof as well as mixtures in different proportions of the separate enantiomers, where such isomers and enantiomers exist, as well as pharmaceutically acceptable salts thereof. Isomers may be separated using conventional techniques, e.g. chromatography or fractional crystallisation. The enantiomers may be isolated by separation of racemate for example by fractional crystallisation, resolution or HPLC. The diastereomers may be isolated by separation of isomer mixtures for instance by fractional crystallisation, HPLC or flash chromatography. Alternatively the stereoisomers may be made by chiral synthesis from chiral starting materials under conditions which will not cause racemisation or epimerisation, or by derivatisation, with a chiral reagent. All stereoisomers are included within the scope of the invention.

### Methods of preparation

The compounds of the invention may be prepared as outlined below. However, the invention is not limited to these methods, the compounds may also be prepared as described for structurally related compounds in the prior art. The reactions can be carried out according to standard procedures or as described in the experimental section.

Compounds of formula I may be prepared by reacting a compound of formula II 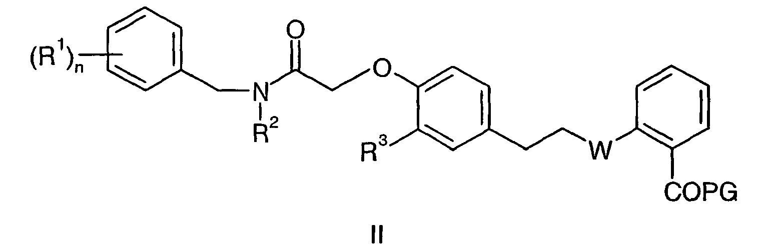 in which R¹, R², R³, W and n are as previously defined and PG represents a protecting group for a carboxylic hydroxy group as described in the standard text "Protective Groups in Organic Synthesis", 2nd Edition (1991) by Greene and Wuts, with a de-protecting agent. The protecting group may also be a resin, such as Wang resin or 2-chlorotrityl chloride resin. Protecting groups may be removed in accordance to techniques which are well known to those skilled in the art. One such protecting group is where PG represents C₁₋₆alkoxy group or an arylalkoxy group eg benzyl, such that COPG represents an ester. Such esters can be reacted with a hydrolysing agent, for example lithium hydroxide in the presence of a solvent for example a mixture of THF and water or potassium hydroxide in a C₁₋₃ alcohol for example methanol, at a temperature in the range of 0-200°C or by microwave radiation to give compounds of formula I.

Compounds of formula II may be prepared by reacting a compound of formula III 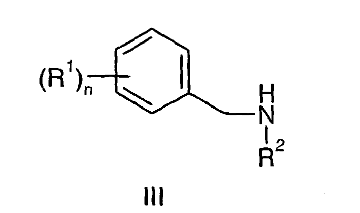 or a salt thereof, for example a hydrochloride salt, in which R¹, R² and n are as previously defined with a compound of formula IV 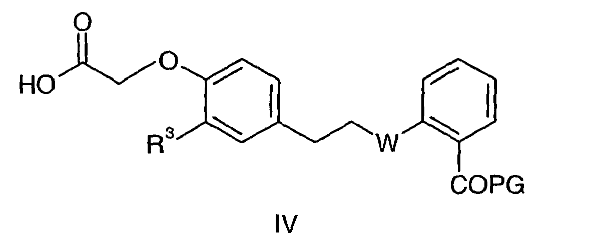 or the acid chloride thereof in which R³ , W and PG are as previously defined in an inert solvent, for example dichloromethane, optionally in the presence of a coupling agent, for example 4-dimethylaminopyridine or 1-ethyl-3-(3-dimethylaminopropyl)carbodiimide hydrochloride, at a temperature in the range of -25°C to 150°C.

Compounds of formula II may also be prepared by reacting a compound of formula V 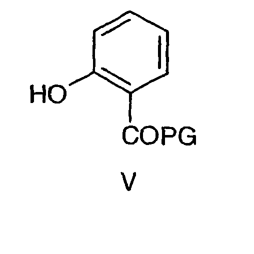 in which PG is as previously defined with a compound of formula VI 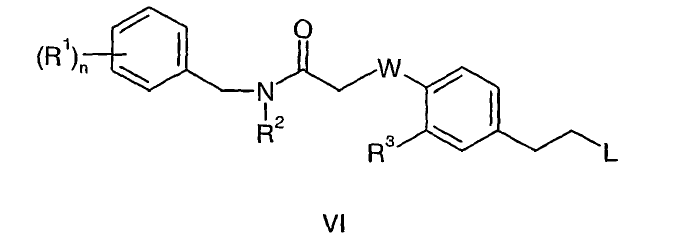 in which R¹, R², R³, W and n are as previously defined and L represents a leaving group, for example methylsulphonyloxy or halo, e.g. bromo, optionally in the presence of solvent, for example acetonitrilie, and optionally in the presence of a base, for example potassium carbonate, at a temperature in the range of 0 to 150°C.

Compounds of formula III , IV, V and VI may be prepared by methods described in the Examples or by analogous methods known to those skilled in the art.

Compounds of formula II, III, IV and V are useful intermediates in the preparation of compounds of formula I. Novel compounds of formula II are herein claimed as a further aspect of the present invention.

The compounds of the invention may be isolated from their reaction mixtures using conventional techniques.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

In any of the preceding methods of preparation, where necessary, hydroxy, amino or other reactive groups may be protected using a protecting group, R^{p} as described in the standard text "Protective groups in Organic Synthesis", 2nd Edition (1991) by Greene and Wuts. The protecting group may also be a resin, such as Wang resin or 2-chlorotrityl chloride resin. The protection and deprotection of functional groups may take place before or after any of the reaction steps described hereinbefore. Protecting groups may be removed in accordance to techniques which are well known to those skilled in the art.

The expression "inert solvent" refers to a solvent which does not react with the starting materials, reagents, intermediates or products in a manner which adversely affects the yield of the desired product.

### Pharmaceutical preparations

The compounds of the invention will normally be administered via the oral, parenteral, intravenous, intramuscular, subcutaneous or in other injectable ways, buccal, rectal, vaginal, transdermal and/or nasal route and/or via inhalation, in the form of pharmaceutical preparations comprising the active ingredient either as a free acid, or a pharmaceutically acceptable salt, in a pharmaceutically acceptable dosage form. Depending upon the disorder and patient to be treated and the route of administration, the compositions may be administered at varying doses.

Suitable daily doses of the compounds of the invention in therapeutical treatment of humans are about 0.0001-100 mg/kg body weight, preferably 0.001-10 mg/kg body weight.

Oral formulations are preferred particularly tablets or capsules which may be formulated by methods known to those skilled in the art to provide doses of the active compound in the range of 0.5mg to 500mg for example 1 mg, 3 mg, 5 mg, 10 mg, 25mg, 50mg, 100mg and 250mg.

According to a further aspect of the invention there is thus provided a pharmaceutical formulation including any of the compounds of the invention, or pharmaceutically acceptable salt thereof, in admixture with pharmaceutically acceptable adjuvants, diluents and/or carriers.

### Pharmacological properties

The present compounds of formula (1) are useful for the prophylaxis and/or treatment of clinical conditions associated with inherent or induced reduced sensitivity to insulin (insulin resistance) and associated metabolic disorders (also known as metabolic syndrome). These clinical conditions will include, but will not be limited to, general obesity, abdominal obesity, arterial hypertension, hyperinsulinaemia, hyperglycaemia, type 2 diabetes and the dyslipidaemia characteristically appearing with insulin resistance. This dyslipidaemia, also known as the atherogenic lipoprotein profile, is characterised by moderately elevated non-esterified fatty acids, elevated very low density lipoprotein (VLDL) triglyceride rich particles, high Apo B levels, low high density lipoprotein (HDL) levels associated with low apoAl particle levels and high Apo B levels in the presence of small, dense, low density lipoproteins (LDL) particles, phenotype B.

The compounds of the present invention are expected to be useful in treating patients with combined or mixed hyperlipidemias or various degrees of hypertriglyceridemias and postprandial dyslipidemia with or without other manifestations of the metabolic syndrome.

Treatment with the present compounds is expected to lower the cardiovascular morbidity and mortality associated with atherosclerosis due to their antidyslipidaemic as well as antiinflammatory properties. The cardiovascular disease conditions include macro-angiopathies of various internal organs causing myocardial infarction, congestive heart failure, cerebrovascular disease and peripheral arterial insufficiency of the lower extremities. Because of their insulin sensitizing effect the compounds of formula I are also expected to prevent or delay the development of type 2 diabetes from the metabolic syndrome and diabetes of pregnancy. Therefore the development of long-term complications associated with chronic hyperglycaemia in diabetes mellitus such as the micro-angiopathies causing renal disease, retinal damage and peripheral vascular disease of the lower limbs are expected to be delayed. Furthermore the compounds may be useful in treatment of various conditions outside the cardiovascular system whether or not associated with insulin resistance, like polycystic ovarian syndrome, obesity, cancer and states of inflammatory disease including neurodegenerative disorders such as mild cognitive impairment, Alzheimer's disease, Parkinson's disease and multiple sclerosis.

The compounds of the present invention are expected to be useful in controlling glucose levels in patients suffering from type 2 diabetes.

The compounds can be used for treating or preventing dyslipidemias, the insulin resistance syndrome and/or metabolic disorders (as defined above) by administration of a compound of formula I to a mammal (particularly a human) in need thereof.

The compounds can be used for treating or preventing type 2 diabetes by administration of an effective amount of a compound of formula I to a mammal (particularly a human) in need thereof.

In a further aspect the present invention provides the use of a compound of formula I for the manufacture of a medicament.

In a further aspect the present invention provides the use of a compound of formula I in the manufacture of a medicament for the treatment of insulin resistance and/or metabolic disorders.

### Combination Therapy

The compounds of the invention may be combined with another therapeutic agent that is useful in the treatment of disorders associated with the development and progress of atherosclerosis such as hypertension, hyperlipidaemias, dyslipidaemias, diabetes and obesity. The compounds of the invention may be combined with another therapeutic agent that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

The compounds of the invention may be used alongside other therapies for the treatment of metabolic syndrome or type 2 diabetes and its associated complications, these include biguanide drugs, for example metformin, phenfonmin and buformin, insulin (synthetic insulin analogues, amylin) and oral antihyperglycemics (these are divided into prandial glucose regulators and alpha-glucosidase inhibitors). An example of an alpha-glucosidase inhibitor is acarbose or voglibose or miglitol. An example of a prandial glucose regulator is repaglinide or nateglinide.

In another aspect of the invention, the compound of formula I, or a pharmaceutically acceptable salt, solvate, solvate of such a salt or a prodrug thereof, may be administered in association with another PPAR modulating agent. PPAR modulating agents include but are not limited to a PPAR alpha and/or gamma and /or delta agonist, or pharmaceutically acceptable salts, solvates, solvates of such salts or prodrugs thereof. Suitable PPAR alpha and/or gamma agonists, pharmaceutically acceptable salts, solvates, solvates of such salts or prodrugs thereof are well known in the art. These include the compounds described in WO 01/12187, WO 01/12612, WO 99/62870, WO 99/62872, WO 99/62871, WO 98/57941, WO 01/40170, J Med Chem, 1996, 39, 665, Expert Opinion on Therapeutic Patents, 10 (5), 623-634 (in particular the compounds described in the patent applications listed on page 634) and J Med Chem, 2000, 43, 527. Particularly a PPAR alpha and/or gamma agonist refers to BMS 298585, clofibrate, fenofibrate, bezafibrate, gemfibrozil and ciprofibrate; GW 9578, pioglitazone, rosiglitazone, rivoglitazone, balaglitazone, KRP-297, JTT-501, SB 213068, GW 1929, GW 7845, GW 0207, L-796449, L-165041 and GW 2433. Particularly a PPAR alpha aud/or gamma agonist refers to (S)-2-ethoxy-3-[4-(2-{4-methanesulphonyloxy-phenyl}ethoxy)phenyl]propauoic acid and pharmaceutically acceptable salts thereof.

In addition the combination of the invention may be used in conjunction with a sulfonylurea for example: glimepiride, glibenclamide (glyburide), gliclazide, glipizide, gliquidone, chloropropamide, tolbutamide, aeetohexamide, glycopyramide, carbutamide, glibonuride, glisoxepid, glybuthiazole, glibuzole, glyhexamide, glymidine, glypinamide, phenbutamide, tolcylamide and tolazamide. Preferably the sulfonylurea is glimepiride or glibenclamide (glyburide). More preferably the sulfonylurea is glimepiride. Therefore the present invention includes administration of a compound of the present invention in conjunction with one, two or more existing therapies described in this paragraph. The doses of the other existing therapies for the treatment of type 2 diabetes and its associated complications will be those known in the art and approved for use by regulatory bodies for example the FDA and may be found in the Orange Book published by the FDA. Alternatively smaller doses may be used as a result of the benefits derived from the combination.The present invention also includes a compound of the present invention in combination with a cholesterol-lowering agent. The cholesterol-lowering agents referred to in this application include but are not limited to inhibitors ofHMG-CoA reductase (3-hydroxy-3-methylglutaryl coenzyme A reductase). Suitably the HMG-CoA reductase inhibitor is a statin selected from the group consisting of atorvastatin, bervastatin, cerivastatin, dalvastatin, fluvastatin, itavastatin, lovastatin, mevastatin, nicostatin, nivastatin, pravastatin and simvastatin, or a pharmaceutically acceptable salt, especially sodium or calcium, or a solvate thereof, or a solvate of such a salt. A particular statin is atorvastatin, or a pharmaceutically acceptable salt, solvate, solvate of such a salt or a prodrug thereof. A more particular statin is atorvastatin calcium salt. A particularly preferred statin is, however, a compound with the chemical name (E)-7-[4-(4-fluorophenyl)-6-isopropyl-2-[methyl(methylsulfonyl)-amino]-pyrimidin-5-yl](3R,55)-3,5-dihydroxyhept-6-enoic acid, [also known as (E)-7-[4-(4-fluorophenyl)-6-isopropyl-2-[*N-*methyl-*N*-(methylsulfonyl)-amino]pyrimidin-5-yl](3R,5S)-3,5-dihydroxyhept-6-enoic acid ] or a pharmaceutically acceptable salt or solvate thereof, or a solvate of such a salt. The compound (E)-7-[4-(4-fluorophenyl)-6-isopropyl-2-[methyl-(methylsulfonyl)-amino]-pyrimidin-5-yl](3R,5S)-3,5-dihydroxyhept-6-enoic acid, and its calcium and sodium salts are disclosed in European Patent Application, Publication No. EP-A-0521471, and in Bioorganic and Medicinal Chemistry, (1997), 5(2), 437-444. This latter statin is now known under its generic name rosuvastatin.

In the present application, the term "cholesterol-lowering agent" also includes chemical modifications of the HMG-CoA reductase inhibitors, such as esters, prodrugs and metabolites, whether active or inactive.

The present invention also includes a compound of the present invention in combination with a bile acid sequestering agent, for example colestipol or cholestyramine or cholestagel.

The present invention also includes a compound of the present invention in combination with an inhibitor of the ileal bile acid transport system (IBAT inhibitor).

Suitable compounds possessing IBAT inhibitory activity have been described, see for instance the compounds described in WO 93/16055, WO 94/18183, WO 94/18184, WO 94/24087, WO 96/05188, WO 96/08484, WO 96/16051, WO 97/33882, WO98/07749, WO 98/38182, WO 98/40375, WO 98/56757, WO 99/32478, WO 99/35135, WO 99/64409, WO 99/64410, WO 00/01687, WO 00/20392, WO 00/20393, WO 00/20410, WO 00/20437, WO 01/34570, WO 00/35889, WO 00/47568, WO 00/61568, WO 01/68637, WO 01/68096, WO 02/08211, WO 00/38725, WO 00/38726, WO 00/38727, WO 00/38728, WO 00/38729, DE 19825804, JP 10072371, US 5070103, EP 251 315, EP 417 725, EP 489 423, EP 549 967, EP 573 848, EP 624 593, EP 624 594, EP 624 595, EP 869 121, EP 864 582, and EP 1 070 703.

Particular classes of IBAT inhibitors suitable for use in the present invention are benzothiepines, and the compounds described in the claims, particularly claim 1, of WO 00/01687, WO 96/08484 and WO 97/33882. Other suitable classes of IBAT inhibitors are the 1,2-benzothiazepines, 1,4-benzothiazepines and 1,5-benzothiazepines. A further suitable class of IBAT inhibitors is the 1,2,5-benzothiadiazepines.

One particular suitable compound possessing IBAT inhibitory activity is (3*R*,5*R*)-3-butyl-3-ethyl-1,1-dioxido-5-phenyl-2,3,4,5-tetrahydro-1,4-benzothiazepin-8-yl β-D-glucopyianosiduronic acid (EP 864 582). Other suitable IBAT inhibitors include one of:
1,1-dioxo-3,3-dibutyl-5-phenyl-7-methylthio-8-(*N*-{(R)-1'-phenyl-1'-[*N*'-(carboxymethyl) carbamoyl]methyl}carbamoylmethoxy)-2,3,4,5-tetrahydro-1,5-benzothiazepine;
1,1-dioxo-3,3-dibutyl-5-phenyl-7-methylthio-8-(*N*-{(*R*)-α-[*N*'-(carboxymethyl)carbamoyl]-4-hydroxybenzyl}carbamoylmethoxy)-2,3,4,5-tetrahydro-1,5-benzothiazepine;
1,1-dioxo-3,3-dibutyl-5-phenyl-7-methylthio-8-(*N*-{(R)-1'-phenyl-1'-[*N'*-(2-sulphoethyl)carbamoyl]methyl}carbamoylmethoxy)-2,3,4,5-tetrahydro-1,5-benzothiazepine;
1,1-dioxo-3-butyl-3-ethyl-5-phenyl-7-methylthio-8-(*N*-{(R)-1'-phenyl-1'-[*N*'-(2-sulphoethyl)carbamoyl]methyl}carbamoylmethoxy)-2,3,4,5-tetrahydro-1,5-benzothiazepine;
1,1-dioxo-3,3-dibutyl-5-phenyl-7-methylthio-8-(*N*-{(R)-α-[*N*'-(2-sulphoethyl)carbamoyl]-4-hydroxybenzyl}carbamoylmethoxy)-2,3,4,5-tetrahydro-1,5-benzothiazepine;
1,1-dioxo-3-butyl-3-etliyl-5-phenyl-7-methylthio-8-(*N*-{(R)-α-[*N*'-(2-sulphoediyl) carbamoyl]-4-hydroxybenzyl}carbamoyhnethoxy)-2,3,4,5-tetrahydro-1,5-benzothiazepine;
1,1-dioxo-3-butyl-3-ethyl-5-phenyl-7-methylthio-8-(*N*-{(R)*-α-*[*N'*-(2-carboxyethyl)carbamoyl]benzyl}carbamoylmethoxy)-2,3,4,5-tetrahydro-1,5-benzothiazepine;
1,1-dioxo-3,3-dibutyl-5-phenyl-7-methylthio-8-(*N*-{(R)-α-[*N*'-(2-carboxyethyl)carbwnoyl]-4-hydroxybenzyl}carbamoylmethoxy)-2,3,4,5-tetrahydro-1,5-benzothiazepine;
1,1-dioxo-3-butyl-3-ethyl-5-phenyl-7-methylthio-8-(*N*-{(R)-α-[*N*'-(5-carboxypentyl) carbamoyl]benzyl}carbamoylmethoxy)-2,3,4,5-tetrahydro-1,5-benzothiazepine;
1,1-dioxo-3,3-dibutyl-5-phenyl-7-methylthio-8-(*N-*{(R)-α-[*N'*-(2-carboxyethyl)carbamoyl] benzyl}carbamoyhnethoxy)-2,3,4,5-tetrahydro-1,5-benzothiazepine;
1,1-dioxo-3,3-dibutyl-5-phenyl-7-methylthio-8-(*N*-{α-[*N*'-(2-sulphoethyl)carbamoyl]-2-fluorobenzyl}carbainoylmethoxy)-2,3,4,5-tetraliydro-1,5-benzothiazepine;
1,1-dioxo-3-butyl-3-ethyl-5-phenyl-7-methylthio-8-(*N*-{(R)-α-[*N*'-(R)-(2-hydroxy-1-carboxyethyl)carbamoyl]benzyl}carbamoylmethoxy)-2,3,4,5-tetrahydro-1,5-benzothiazepine;
1,1-diuxo-3,3-dlbutyl-5-phenyl-7-methylthio-8-(*N*-{(R)-α-[*N*'-(R)-(2-hydroxy-1-carboxyethyl)carbamoyl]benzyl}carbamoylmethoxy)-2,3,4,5-tetrahydro-1,5-benzothiazepine;
1,1-dioxo-3,3-dibutyl-5-phenyl-7-methylthio-8-{*N*-[(R)-α-(N'-{(R)-1-[*N*"-(R)-(2-hydroxy-1-carboxyethyl)carbamoyl]-2-hydroxyethyl}carbamoyl)benzyl]carbamoylmethoxy}-2,3,4,5-tetrahydro-1,5-benzothiazepine;
1,1-dioxo-3-butyl-3-ethyl-5-phenyl-7-methylthio-8-(*N*-{α-[*N*'-(carboxynletliyl)carbamoyl] benzyl }carbamoylmethoxy)-2,3,4,5-tetraliydro-1,5-btnzothiazepine;
1,1-dioxo-3-butyl-3-ethyl-5-phenyl-7-methylthio-8-(*N*-{α-[*N'-*((ethoxy)(methyl)phosphoryl-methyl)carbamoyl]benzyl}carbamoylmetlioxy)-2,3,4,5-tetrahydro-1,5-benzothiazepine;
1,1-dioxo-3-butyl-3-ethyl-5-phenyl-7-methylthio-8-{*N*-[(R)-α-(*N*'-{2-[(hydroxy)(methyl)phosphoryl]ethyl}carbamoyl)benzyl]carbamoylmethoxy}-2,3,4,5-tetrahydro-1,5-benzothiazepine;
1,1-dioxo-3,3-dibutyl-5-phenyl-7-methylthio-8-(*N*-{(R)-α-[*N*'-(2-methylthio-1-carboxyethyl)carbamoyl]benzyl}carbamoyhmethoxy)-2,3,4,5-tetrahydro-1,5-benzothiazepine;
1,1-dioxo-3,3-dibutyl-5-phenyl-7-methylthio-8-{*N*-[(R)-α-(*N*'-{2-[(methyl)(ethyl) phosphoryl]ethyl}carbamoyl)-4-hydroxybenzyl]carbamoylmethoxy}-2,3,4,5-tetrahydro-1,5-benzothiazepine;
1,1-dioxo-3,3-dibutyl-5-phenyl-7-methylthio-8-{*N*-[(R)-α-(*N*'-{2-[(methyl)(hydroxy) phosphoryl]ethyl}carbamoyl)-4-hydroxybenzyl]carbamoylmethoxy}-2,3,4,5-tetrahydro-1,5-benzothiazepine;
1,1-dioxo-3,3-dibutyl-5-phenyl-7-mefllylthio-8-(*N*-{(R)-α-[(R)-*N*'-(2-methylsulphinyl-1-carboxyethyl)carbamoyl]benzyl}carbamoylmethoxy)-2,3,4,5-tetrahydro-1,5-benzothiazepine;
1,1-dioxo-3,3-dibutyl-5-phenyl-7-methoxy-8-[*N*-{(R)-α-[*N*'-(2-sulphoethyl)carbamoyl]-4-hydroxybenzyl}carbamoylmethoxy]-2,3,4,5-tetrahydro-1,5-benzothiazepine;
1,1-dioxo-3,3-dibutyl-5-phenyl-7-methylthio-8-(*N*-{(R)-α-[*N*-((R)-1-carboxy-2-methylthio-ethyl)carbamoyl]-4-hydroxybenzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(R)-α-[*N*-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(*R*)-α-[*N*-((S)-1-carboxy-2-metliylpropyl)carbamoyl]-4-hydroxybenzyl}carbamoyhnethoxy)-2,3,4,5-tetrahydro-1,2,5-benzothiadiazepine;
1,1-dioxo-3,3-dibutyl-5-plienyl-7-methylthio-8-(*N*-{(R)-α-[*N*-((S)-1-carboxybutyl) carbamoyl]-4-hydroxybenzyl}carbamoylmethoxy)-2,3,4,5-tetraliydro-1,2,5-benzothiadiazepine;
1,1-dioxo-3,3-dibutyl-5-phenyl-7-methylthio-8-(*N*-{(R)-α-[*N*-((S)-1-carboxypropyl) carbamoyl]benzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(R)-α-[*N*-((S)-1-carboxyethyl) carbwnoyl]benzyl}carbwnoylmethoxy)-2,3,4,5-tetroydro-1,2,5-benzothiadiazepine;
1,1-dioxo-3,3-dibutyl-5-phenyl-7-methylthio-8-(*N*-{(R)-α-[*N*-((S)-1-carboxy-2-(R)-hydroxypropyl)carbamoyl]benzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(R)-α-[*N*-(2-sulphoethyl)carbamoyl]-4-hydroxybenzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(R)-α-[*N*-((S)-1-carboxyethyl)carbamoyl]-4-hydroxybenzyl}carbamoylmethoxy)-2,3,4,5-tetrahydro-1,2,5-benzothiadiazepine;
1,1-dioxo-3,3-dibutyl-5-phenyl-7-methylthio-8-(*N-*{(R)*-α-*[*N-*((R)-1-carboxy-2-methylthioethyl)carbamoyl]benzyl}carbamoylmethoxy)-2,3,4,5-tetrahydro-1,2,5-benzothiadiazepine;
1,1-dioxo-3,3-dibutyl-5-phenyl-7-methylthio-8-(*N*-{(R)-α-[*N*-{(S)-1-[*N*-((S)-2-hydroxy-1-carboxyethyl)carbamoyl]propyl}carbamoyl]benzyl}carbamoyhnethoxy)-2,3,4,5-tetrahydro-1,2,5-benzothiadiazepine
1,1-dioxo-3,3-dibutyl-5-phenyl-7-methylthio-8-(*N*-{(R)-α-[*N*-((S)-1-carboxy-2-methylpropyl)carbamoyl]benzyl}carbamoylmethoxy)-2,3,4,5-tetrahydro-1,2,5-benzothiadiazepine;
1,1-Dioxo-3,3-dibutyl-5-phenyl-7-methylthio-8-(*N*-{(R)-α-[*N*-((S)-1-carboxypropyl) carbamoyl]-4-hydroxybenzyl}carbamoylmethoxy)-2,3,4,5-tetrahydro-1,2,5-benzothiadiazepine;
1,1-Dioxo-3,3-dibutyl-5-phenyl-7-methylthio-8-[*N*-(R/S)-α-{*N*-[1-(R)-2-(S)-1-hydroxy-1-(3,4-dihydroxyphenyl)prop-2-yl]carbamoyl}-4-hydroxybenzyl)carbamoylmethoxy]-2,3,4,5-tetrahydro-1,2,5-benzothiadiazepine;
1,1-Dioxo-3,3-dibutyl-5-phenyl-7-methylthio-8-(*N*-{(R)-α-[*N*-(2-(S)-3-(R)-4-(R)-5-(R)-2,3,4,5,6-pentahydroxyhexyl)carbamoyl]-4-hydroxybenzyl }carbamoylmethoxy)-2,3,4,5-tetrahydro-1,2,5-benzothiadiazepine; and
1,1-Dioxo-3,3-dibutyl-5-phenyl-7-methylthio-8-(*N*-{(R)-α-[*N*-(2-(S)-3-(R)-4-(R)-5-(R)-2,3,4,5,6-pentahydroxyhexyl)carbatnoyl]benzyl}carbamoylmethoxy)-2,3,4,5-tetrahydro-1,2,5-benzothiadiazepine;
or a pharmaceutically acceptable salt, solvate, solvate of such a salt or a prodrug thereof.

According to an additional further aspect of the present invention there is provided a combination treatment comprising the administration of an effective amount of a compound of the formula I, or a pharmaceutically acceptable salt, solvate, solvate of such a salt or a prodrug thereof, optionally together with a pharmaceutically acceptable diluent or carrier, with the simultaneous, sequential or separate administration one or more of the following agents selected from
a CETP (cholesteryl ester transfer protein) inhibitor, for example those referenced and described in WO 00/38725 page 7 line 22 - page 10, line 17;
a cholesterol absorption antagonist for example azetidinones such as SCH 58235 and those described in US 5,767,115;
a MTP (microsomal transfer protein) inhibitor for example those described in Science, 282, 751-54, 1998;
a nicotinic acid derivative, including slow release and combination products, for example, nicotinic acid (niacin), acipimox and niceritrol;
a phytosterol compound for example stanols;
probucol;
an anti-obesity compound for example orlistat (EP 129,748) and sibutramine (GB 2,184,122 and US 4,929,629);
an omega-3 fatty acid for example Omacor^{™};
an antihypertensive compound for example an angiotensin converting enzyme (ACE) inhibitor, an angiotensin II receptor antagonist, an andrenergic blocker, an alpha andrenergic blocker, a beta andrenergic blocker for example metoprolol, a mixed alpha/beta andrenergic blocker, an andrenergic stimulant, calcium channel blocker, an AT-1 blocker, a saluretic, a diuretic or a vasodilator;
a CB 1 antagonist or inverse agonist for example as described in WO01/70700 and EP 65635 ;
aspirin;
a Melanin concentrating hormone (MCH) antagonist;
a PDK inhibitor; or
modulators of nuclear receptors for example LXR, FXR, RXR, and RORalpha;
or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

Particular ACE inhibitors or pharmaceutically acceptable salts, solvates, solvate of such salts or a prodrugs thereof, including active metabolites, which can be used in combination with a compound of formula I include but are not limited to, the following compounds: alacepril, alatriopril, altiopril calcium, ancovenin, benazepril, benazepril hydrochloride, benazeprilat, benzoylcaptopril, captopril, captopril-cysteine, captopril-glutathione, ceranapril, ceranopril, ceronapril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, glycopril, hemorphin-4, idrapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, utibapril, zabicipril, zabiciprilat, zofenopril and zofenoprilat. Preferred ACE inhibitors for use in the present invention are ramipril, ramiprilat, lisinopril, enalapril and enalaprilat. More preferred ACE inhibitors for uses in the present invention are ramipril and ramiprilat.

Preferred angiotensin II antagonists, pharmaceutically acceptable salts, solvates, solvate of such salts or a prodrugs thereof for use in combination with a compound of formula I include, but are not limited to, compounds: candesartan, candesartan cilexetil, losartan, valsartan, irbesartan, tasosartan, telmisartan and eprosartan. Particularly preferred angiotensin II antagonists or pharmaceutically acceptable derivatives thereof for use in the present invention are candesartan and candesartan cilexetil.

The compounds of the invention can be used for the treatment of type 2 diabetes and its associated complications in a warm-blooded animal, such as man, in need of such treatment which comprises administering to said animal an effective amount of a compound of formula I, or a pharmaceutically acceptable salt, solvate, or solvate of such a salt in simultaneous, sequential or separate administration with an effective amount of one of the other compounds described in this combination section, or a pharmaceutically acceptable salt, solvate, solvate of such a salt or a prodrug thereof.

The compounds can also be used for treating hyperlipidemic conditions in a warm-blooded animal, such as man, in need of such treatment by administering to said animal an effective amount of a compound of formula I, or a pharmaceutically acceptable salt, solvate, or solvate of such a salt in simultaneous, sequential or separate administration with an effective amount of one of the other compounds described in this combination section or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt, solvate, or solvate of such a salt, and one of the other compounds described in this combination section or a pharmaceutically acceptable salt, solvate, solvate of such a salt or a prodrug thereof, in association with a pharmaceutically acceptable diluent or carrier.

According to another feature of the invention there is provided the use of a compound of the formula I, or a pharmaceutically acceptable salt, solvate, solvate of such a salt or a prodrug thereof, and one of the other compounds described in this combination section, or a pharmaceutically acceptable salt, solvate, or solvate of such a salt, in the manufacture of a medicament for use in the the treatment of metabolic syndrome or type 2 diabetes and its associated complications in a warm-blooded animal, such as man.

According to another feature of the invention there is provided the use of a compound of the formula I, or a pharmaceutically acceptable salt, solvate, or solvate of such a salt and one of the other compounds described in this combination section, or a pharmaceutically acceptable salt, solvate, solvate of such a salt or a prodrug thereof, in the manufacture of a medicament for use in the treatment of hyperlipidaemic conditions in a warm-blooded animal, such as man.

### Working examples

¹H NMR and ¹³C NMR measurements were performed on a Varian Mercury 300 or Varian UNITY plus 400, 500 or 600 spectrometers, operating at ¹H frequencies of 300, 400, 500 and 600 MHz, respectively, and at ¹³C frequencies of 75, 100, 125 and 150 MHz, respectively. Measurements were made on the delta scale (δ).

Unless otherwise stated, chemical shifts are given in ppm with the solvent as internal standard.

### Abbreviations

- IRS: insulin resistance syndrome
- TLC: thin layer chromatography
- HOBT: 1-hydroxybenzotriazole-hydrate
- DIBAH: diisobutylaluminium hydride
- DMSO: dimethyl sulfoxide
- EtOAc: ethyl acetate
- DMF: *N,N*-dimethylformamide
- THF: tetrahydrofuran
- HPLC: high performance liquid chromatography
- MeCN: acetonitrile
- TFA: trifluoroacetic acid
- Pd/C: palladium on charcoal
- HATU: O-(7-azabenzotriazolyl-1- yl)-N,N,N',N'-tetramethyluronium hexafluorophosphate
- DCM: dichloromethane
- TBTU: O-(benzotriazol-1- yl)-N,N,N',N'-tetramethyluronium tetrafluoroborate
- DIPEA: *N,N*-diisopropylethylamine
- DMAP: 4-dimethylaminopyridine
- Trisamine: Tris(hydroxymethyl)aminomethane

ISOLUTE ® FLASH Si is a silica column suitable for chromatography Borohydride on polymer support is Borohydride on Amberlite IRA-400 available from Aldrich
- LC-MS: liquid chromatography- mass spectroscopy
- RT: room temperature
- t: triplet
- s: singlet
- d: doublet
- q: quartet
- qvint: quintet
- m: multiplet
- br: broad
- bs: broad singlet
- dm: doublet of multiplet
- bt: broad triplet
- dd: doublet of doublets

### Example 1

### a) Tert-butyl [4-(2-hydroxyethyl)phenoxy]acetate

A mixture of 4-(2-hydroxyethyl)phenol (3.8ml, 25.834mmol) was dissolved in acetonitrile (25ml), potassium carbonate (7.085g, 51.267rnmol) and *tert*-butyl bromoacetate (5.000g, 25.834mmol) was boiled under reflux for 16 hours. The solvent was evaporated under reduced pressure. The residue was dissolved in EtOAc and washed with brine and water, dried with MgSO₄ and evaporated under reduced pressure to give the desired product was obtained (6.00g , yield 92.8 %)
¹H-NMR (400MHz, CDCl₃): 1.52 (s, 9H), 2.98 (t, 2H), 3.46 (t, 2H), 4.92 (s, 2H), 6.89-6.97 (m, 4H)

### b) Tert-butyl (4-{2-[(methylsulfonyl)oxy]ethyl}phenoxy)acetate

*Tert*-butyl [4-(2-hydroxyethyl)phenoxy]acetate (6.000g, 23.781mmol) and triethylamine (9.9ml, 71.341mmol) were dissolved in DCM. The mixture was cooled to -10°C and methanesulfonyl chloride (2.8ml, 35.671mmol) was added dropwise to the mixture. The reaction mixture was allowed to reach room temperature and was stirred for 16 hours. The mixture was diluted with DCM. The organic layer was washed with water, brine and 0.3M KHSO₄, dried with MgSO₄, and evaporated under reduced pressure. Obtained 7.5g of light-yellow crystals (yield 95.5 %).
¹H-NMR (400MHz, CDCl₃): 1.52 (s, 9H), 2.98 (t, 2H),3.10(s, 3H), 3.46 (t, 2H), 4.92 (s, 2H), 6.89-6.97 (m, 4H)

### c) Methyl 2-{2-[4-(2-tert-butoxy-2-oxoethoxy)phenyl]ethoxy}benzoate

Methyl salicylate (2.7ml, 21.187mmol) was dissolved in acetonitrile, and potassium carbonate (5.856g, 42.373mmol) was added. The mixture was cooled to -10°C then *tert-*butyl (4-{2-[(methylsulfonyl)oxy]ethyl}phenoxy)acetate was added. The mixture was boiled under reflux for 16 hours, and then the solvent was evaporated under reduced pressure. The residue was dissolved in EtOAc, washed with water and brine, then the organic layer was dried with MgSO₄ and the solvent was removed by evaporation. The crude material was purified by flash chromatography (silica gel 60 0.004-0.063mm) using EtOAc: Toluene 50:50 as the eluant. The fractions which contained the desired product were pooled, and solvent evaporated. This gave 5.0g of pure product (yield 61.1 %).
¹H-NMR (400MHz, CDCl₃); 1.48 (s, 9H), 3.08 (s, 3H), 3.87 (t, 2H), 4.18 (t, 2H), 4.49 (s, 2H), 6.84 (d, 2H), 6.90-6.98 (m, 2H), 7.20-7.26 (m, 2H), 7.38-7.43 (m,1H), 7.7 (dd, 1H)

### d) (4-{2-[2-(methoxycarbonyl)phenoxy]ethyl}phenoxy)acetic acid

Methyl 2-{2-[4-(2-*tert*-butoxy-2-oxoethoxy)phenyl]ethoxy}benzoate (0.400 g, 1.0351mmol) was dissolved in DCM and trifluoracetic acid (0.8ml, 8.281mmol) was added. The mixture was stirred at room temperature for 3h. The solvent was evaporated to give 325 mg of a white powder.
¹H-NMR (600MHz, CDCl₃); 3.08 (t, 2H), 3.86 (s, 3H), 4.18 (t, 2H), 4.64 (s, 2H), 6.84-6.96 (m, 4H), 7,23 (d, 2H), 7.37-7.42 (m, 1H), 7.75 (dd, 1H)

### e) Methyl 2-[2-(4-{2-[ethyl(2-fluorobenzyl)amino]-2-oxoethoxy}phenyl)ethoxyl]-benzoate

(4-{2-[2-(Methoxycarbonyl)phenoxy]ethyl}phenoxy)acetic acid (0.200mg, 0.605 mmol) was dissolved in DMF and cooled on an ice-bath. *N*-(2-Fluorobenzyl) ethanamine (0.102 g, 0.666mmol), TBTU (0.214 g, 0.666 mmol) and DIPEA (0.22 ml, 1.271 mmol) was added. The reaction mixture was stirred for 16h at room temperature.EtOAc was added and the organic phase was washed with two portions of 20ml NaCO₃ (sat). The organic layer was dried with MgSO₄ and the solvent was removed by evaporation. The crude was purified by preparative HPLC (starting with acetonitrile/buffer 60/40 and then increasing the acetonitrile concentration to 100% acetonitrile in 25 min, the buffer was a mixture of acetonitrile/water 10/90 and ammonium acetate (0.1 M), column KR-100-7-C8, 50*500, flow 80ml/min). 145 mg of the desired product was obtained after freeze drying ( yield 71.1 %)
¹H-NMR (400MHz, CD3CO) (rotamers); 1.08, 1.17 (t, t, 3H), 2.96 (s, 3H), 3.07 (m, 2H), 3.31, 3.36 (m, 2H), 4.21 (m, 2H), 4.85 (s, 2H), 4.56-4.82 (m, 2H), 6.18 (d, 1H), 6.88-7.06 (m, 3H), 7.18 -7.35 (m, 6H), 7.42 (m, 1H), 7.70 (d, 1H)

### f) 2-[2-(4-{2-[ethyl(2-fluorobenzyl)amino]-2-oxoethoxy}phenyl)ethoxy]benzoic acid

Methyl 2-[2-(4-{2-[ethyl(2-fluorobenzyl)amino]-2-oxoethoxy}phenyl)ethoxy] benzoate (0.200g, 0.115mmol) was dissolved in 3ml THF in a Smith synthesiser vial and then 1.5 ml water and lithium hydroxide (0.032 g, 1.335mmol) were added to the vial. The vial was capped and put in the microwave oven (Smith synthesiser). The reaction was then heated to 150°C for 6 minutes. According to LC-MS the reaction was complete. The solvent was evaporated. The residue was dissolved in diethyl ether (30 ml) and washed with NaHCO₃ (sat) (2x20ml). The basic water layer was acidified to pH 1 with 2M HCl. The water layer was extracted with three portions of 20 ml of DCM which were combined, dried and evaporated to give 160 mg of pure desired product.
¹H-NMR (400MHz, CD3CO) (rotamers); 1.07,1.15 (t, t, 3H), 3.10 (m, 2H), 3.30, 3.36 (m, m, 2H), 4.21 (m, 2H), 4.55-4.67 (m, 2H), 4.90 (s, 2H), 6.20 (d,1H), 6.87-7.06 (m, 3H), 7.18-7.35 (m, 6H), 7.40(m, 1H), 7.70 (d, 1H)

### Example 2

### a) 2-Bromo-N-(2,4-difluorobenzyl)-N-heptylacetamide

*N*-(2,4-difluorobenzyl)-*N*-heptylamine (2.004 g, 8.304 mmol) was dissolved in DCM (30 ml). It was then cooled in an ice-bath. Triethylamine (1.092 g, 10.796 mmol) was added and then bromacetyl chloride (1.438 g, 9.135 mmol) was dropped in. The mixture was stirred for 2 hours (ice-bath). It was then washed with water (with additional of 1% hydrochloric acid, pH-3), water and brine, and dried (magnesium sulphate) and evaporated. The crude oil product was dissolved in DCM, then loaded onto a column (ISOLUTE®SI 5g/25 ml) and eluted with more DCM. Oil product 2.412 g was obtained, yield 80%
¹H NMR (rotamer, 500 MHz, CDCl₃): δ 0.88-0.93 (m, 3H), 1.27-1.34 (m, 8H), 1.52-1.68 (m, 2H), 3.28-3.35 (m, 2H), 3.90-4.15 (m, 2H), 4.61, 4.63 (s, s, 2H), 6.81-6.94 (m, 2H) and 7.15-7.20, 7.34-7.39 (m, 1H).

### b) N-(2,4-difluorobenzyl)-N-heptyl-2-[4-(2-hydroxyethyl)-2-methoxyphenoxy]acetamide

2-Bromo-*N*-(2,4-difluorobenzyl)-*N*-heptylacetamide (135 mg, 0.373 mmol), homovanillyl alcohol (63 mg, 0.373 mmol) and potassium carbonate anhydrous (77 mg, 0.559 mmol) were mixed in acetonitrile (10 ml). The mixture was heated to reflux for 4 hours and then evaporated to dryness. The residue (with additional DCM, 1ml x2) was loaded onto a column (ISOLUTE® SI, lg/6ml). It was eluted with DCM and then MeOH/DCM (0.5:99.5, then 1:99). The product fractions were combined and evaporated. Oil product 132 mg was obtained yield 79%.
¹H NMR (rotamer, 400 MHz, CDCl₃): δ 0.82-0.87 (m, 3H), 1.17-1.28 (m, 8H), 1.43-1.68 (m, 2H), 2.75-2.80 (m, 2H), 3.24-3.32 (m, 2H), 3.73-3.84 (m, 5H), 4.58, 4.66 (s, s, 2H), 4.74, 4.76 (s, s, 2H), 6.67-6.86 (m, 5H) and 7.08-7.14, 7.23-7.29 (m, 1H).

### c) 2-(4-{2-[(2,4-Difluorobenzyl)(heptyl)amino]-2-oxoethoxy}-3-methoxyphenyl)ethyl methanesulfonate

*N*-(2,4-difluorobeuzyl)-*N*-heptyl-2-[4-(2-hydroxyethyl)-2-methoxyphenoxy]acetamide (A)(132 mg, 0.294 mmol) was dissolved in DCM (10 ml). It was cooled in an ice-bath Triethylamine (0.05 ml, 0.352 mmol) was added and then methanesulfonyl chloride (37 mg, 0.323 mmol) was dropped in. The cooling-bath was removed after 30 minutes. The mixture was stirred at room temperature overnight. LS-MS showed that ca 50% of A was not reacted. The mixture was cooled in an ice-bath and 0.05 ml of triethylamine was added, followed by 0.025 ml of methanesulfonyl chloride. After addition, the cooling-bath was removed and the mixture was stirred for 5 hours more. It was then washed with water (x2) and brine, dried (magnesium sulphate) and evaporated. Oil product 138 mg was left and used for next step without further purification.

### d) Methyl 2-[2-(4-{2-[(2,4-difluorobenzyl)(heptyl)amino]-2-oxoethoxy}-3-methoxyphenyl)ethoxy]benzoate

2-(4-{2-[(2,4-Difluorobenzyl)(heptyl)amino]-2-oxoethoxy}-3-mnethoxyphenyl)ethyl methanesulfonate (138 mg, 0.262 mmol) was dissolved in acetonitrile (10 ml). 2-Hydroxybenzoic acid methyl ester (40 mg, 0.262 mmol) was added and then potassium carbonate anhydrous (54 mg, 0.392 mmol) was added. The mixture was heated to reflux overnight and then evaporated to dryness. Water (10 ml) and ethyl acetate (10 ml) were added and the two phases were separated. The organic phase was washed with water and brine, dried (magnesium sulphate) and evaporated. Chromatography of the residue on a column (ISOLUTE® SI, 2 g/ 6 ml) using DCM, MeOH/DCM (1:99) as eluant gave 78 mg the desired product, yield 45% (two steps).
¹H NMR (rotamer, 500 MHz, CDCl₃): δ 0.87-0.91 (m, 3H), 1.22-1.32 (m, 8H), 1.48-1.63 (m, 2H), 3.09-3.14 (m, 2H), 3.28-3.35 (m, 2H), 3.80, 3.89 (s, s, 3H), 3.89 (s, 3H), 4.21-4.25 (m, 2H), 4.62, 4.71 (s, s, 2H), 4.79, 4.81 (s, s, 2H), 6.77-7.01 (m, 7H), 7.28-7.33 (m, 1H), 7.13-7.18, 7.28-7.33 (m, m, 1H), 7.45 (t, 1H) and 7.81 (d, 1H).

### e) 2-[2-(4-{2-[(2,4-Difluorobenzyl)(heptyl)amino]-2-oxoethoxy}-3-methoxyphenyl)ethoxy]benzoic acid

Methyl2-[2-(4-{2-[(2,4-difluorobenzyl)(heptyl)amino]-2-oxoethoxy}-3-methoxyphenyl)-ethoxy]benzoate (74 mg, 0.127 mmol) dissolved in THF (2 ml) was mixed with lithium hydroxide (6.1 mg, 0.254 mmol) dissolved in water (1 ml). The mixture was irradiated in a microwave oven (Smith Synthesizer) at 150°C for 8 minutes. LC-MS showed that the reaction was not complete. It was in the oven for additional 10 minutes, LC-MS showed almost no change. 3 mg more of lithium hydroxide was added and thereafter it was in the oven at 150°C for 8 minutes. LC-MS showed it was still the same as before. 3 mg more of lithium hydroxide and 1 ml water was added. The resulting mixture was in the oven at 150 °C for 10 minutes and LC-MS showed the reaction was complete. It was evaporated to remove THF. The residue was acidified with 1% hydrochloric acid, pH~5, and extracted with ethyl acetate (10 ml). The extracts was dried (magnesium sulphate) and evaporated. Chromatography of the residue on a column (ISOLUTE® SI, 1 g/ 6 ml) using DCM and then MeOH/DCM (1:99) as eluant gave 60 mg the desired product, yield 83%.
¹H NMR (rotamer, 400 MHz, CDCl₃): δ 0.82-0.87 (m, 3H), 1.18-1.28 (m, 8H), 1.43-1.61 (m, 2H), 3.10-3.15(m, 2H), 3.24-3.31 (m, 2H), 3.77, 3.85 (s, s, 3H), 4.39-4.44 (m, 2H), 4.59, 4.66 (s, s, 2H), 4.77, 4.75 (s, s, 2H), 6.72-6.91 (m, 5H), 7.01 (d, 1H), 7.09 (t, 1H), 7.10-7.17, 7.26-7.32 (m, m, 1H), 7.51 (t, 1H) and 8.13 (d, 1H).
¹³C NMR (rotamers, 75 MHz, CDCl₃): δ 14.07, 22.55, 26.79, 27.02, 28.57, 28.93, 31.70, 35.18, 41.30, 41.34, 44.02, 45.89, 46.99, 55.71, 55.82, 68.19, 68.94, 70.66, 103.38(t), 103.88(t), 111.35(d), 111.39(d), 112.32, 112.41, 112.46, 114.76, 117.64, 119.60(dd), 120.07(dd), 120.53, 122.06, 129.50(dd), 130.54, 130.59, 131.55(dd), 133.60, 134.78, 146.26, 146.39, 149.67, 157.10, 161.60(dd), 160.68(dd), 161.97(dd), 162.24(dd), 165.12, 167.75 and 167.93.

### Example 3

### a) N-(4-chlorobenzyl)acetamide

Acetic acid (1.321g, 22.000 mmol) was dissolved in DMF (10 ml), 1-(4-chlorophenyl)methanamine (2.804 g, 19.800 mmol) was added and the mixture was cooled to 0° C. *N*-[(1*H*-1,2,3-benzotriazol-1-yloxy)(dimethylamino)methylene]-*N-*methylmethanaminium tetrafluoroborate (7.770 g, 24.200 mmol) and *N-*ethyl*-N,N-*diisopropylamine (5.971 g, 46.200 mmol) was added. The solution was stirred for two hours at room temperature. EtOAc (20 ml) was added and the organic phase was washed with Na₂CO₃ (3 X 20 ml, aq) and HCl (0.5 M, 2 X, 10 ml). The organic layer was dried (MgSO₄) and the solvent was removed by evaporation. The residue was purified by preparative HPLC (the initial mobile phase was isocratic acetonitrile/buffer 60/40 and then the acetonitrile concentration was increased to 100%, the buffer was a mixture of acetonitrile/water 10/90 and ammonium acetate (0.1 M, column KR-100-7-C8,50 mmX 250 mm, flow 40 ml/min). The product-containing fractions were pooled and the acetonitrile was removed by evaporation. EtOAc (10 ml) was added and the organic phase was washed with two portions of brine and dried (MgSO₄) and the solvent was removed by evaporation and gave 2.337 g of *N*-(4-chlorobenzyl)acetamide (yield 57.8%).
¹HNMR (500 MHz, CDCl₃): δ 1.96 (s, 3H), 4.31 (d, 2H), 6.46 (bs, 1H), 7.16 (d, 2H), 7.25 (d, 2H).

### b) N-(4-chlorobenzyl)-N-ethylamine

*N*-(4-chlorobenzyl)acetamide (2.337 g, 12.726 mmol) was dissolved in THF (100 ml) and was cooled to zero degrees under argon atmosphere. (Methylthio)methane compound with borane (1:1) (2.417 g, 31.815 mmol) was added and the mixture was refluxed overnight at RT. HCl (15 ml, 10%) was gently added and was stirred overnight. The solvent was removed by evaporation. Diethyl ether (20 ml) was added and the product was extracted to the water phase by K₂CO₃ (3 X 15 ml). The aqueous phase was acidified by HCl (10 ml, 10%) and the product was extracted to the organic phase by EtOAc (3 X 15 ml). The organic phase was dried (MgSO₄) and the solvent was removed by evaporation to give 0.938 g of *N*-(4-chlorobenzyl)-*N*-ethylamine (yield 43.4%).
¹HNMR (500 MHz, CDCl₃): δ 1.11 (t, 3H), 2.65 (q, 2H), 3.74 (s, 2H), 7.23-7.28 (m, 4H).

### c) Methyl 2-({2-[4-(2-tert-butoxy-2-oxoethoxy)phenyl]ethyl}thio)benzoate

*Tert*-butyl (4-{2-[(methylsulfonyl)oxy]ethyl}phenoxy)acetate (5.454 g, 17.347 mmol) was dissolved in acetonitrile (100 ml), methyl 2-mercaptobenzoate (3.502 g, 20.816 mmol) and potassium carbonate (4.795 g, 34.694 mmol) was added. The solution was stirred for 10 hours at 60 °C. EtOAc (40 ml) was added and the organic phase was washed with two portions of Brine (2 X 40 ml, aq). The organic layer was dried (MgSO₄ and the solvent was removed by evaporation to give 6.931 g of methyl 2-({2-[4-(2-*tert*-butoxy-2-oxoethoxy)phenyl]ethyl}thio)benzoate. This material was used in the next step without further purification.

### d) [4-(2-{[2-(Methoxycarbonyl)phenyl]-thio}ethyl)-phenoxy]acetic acid

Methyl 2-({2-[4-(2-*tert*-butoxy-2-oxoethoxy)phenyl]ethyl}thio)benzoate (4.630 g, 11.502 mmol) was taken up into DCM (50 ml) and treated with trifluoroacetic acid (44.40 g, 389.405 mmol) at RT for 4h. The mixture was evaporated and azeotroped with toluene. The crude was purified by preparative HPLC (the initial mobile phase was isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. EtOAc (10 ml) was added and the organic phase was washed with two portions of brine and dried (MgSO₄). The solvent was removed by evaporation to give 3.825 g of [4-(2-{[2-(methoxycarbonyl)phenyl]-thio }ethyl)-phenoxy]acetic acid (yield for two steps 63.9% overall).
¹HNMR (500 MHz, CDCl₃): δ 2.93-2.98 (m, 2H), 3.12-3.17 (m, 2H), 3.92 (s, 3H), 4.67 (s, 2H), 6.88 (d, 2H), 7.13-7.21 (m, 3H), 7.33 (d, 1H), 7.41-7.46 (m, 1H), 7.96 (dd, 1H).

### e) Methyl 2-{[2-(4-{2-[(4-chlorobenzyl)(ethyl)amino]-2-oxoethoxy}phenyl)-ethyl]thio}benzoate

[4-(2-{[2-(Methoxycarbonyl)phenyl]thio}ethyl)phenoxy]acetic acid (0.200 g, 0.577 mmol) was dissolved in DMF (10 ml), *N*-(4-chlorobenzyl)-*N*-ethylamine (0.108 g, 0.635 mmol) was added and the mixture was cooled to 0° C. *N*-[(1*H*-1,2,3-benzotriazol-1-yloxy)(dimethylamino)methylene]-*N*-methylmethanaminium tetrafluoroborate (0.204 g, 0.635 mmol) and *N*-ethyl-*N*,*N*-diisopropylamine (0.157 g, 1.212 mmol) were added. The solution was stirred overnight at room temperature. Water (100 ml) was added and the water phase was extracted with diethyl ether (3 X 20ml). The organic phase was washed with Na₂CO₃ (3 X 20 ml, aq) and HCl (0.5 M, 2 X, 10 ml). The organic layer was dried (MgSO₄) and the solvent was removed by evaporation. The residue was purified by flash chromatography (started with isocratic heptane/EtOAc 30/70 and then the EtOAc concentration was increased to 100%, (silica gel 60 0.004-0.063 mm). The product containing fractions were pooled and the solvent was removed by evaporation to give 0.085 g of methyl2-{[2-(4-{2-[(4-chlorobenzyl)(ethyl)amino]-2-oxoethoxy}phenyl)ethyl]thio}benzoate (yield 29.6%).
¹HNMR (rotamers, 300 MHz, CDCl₃): δ 1.09-1.21 (m, 3H), 2.91-2.99 (m, 2H), 3.11-3.18 (m, 2H), 3.32-3.43 (m, 2H), 3.92 (s, 3H), 4.57-4.75 (m, 4H), 6.78, 6.92 (d, d, 2H), 7.12-7.46 (m, 9H), 7.96 (d, 1H).

### f) 2-{[2-(4-{2-[(4-chlorobenzyl)(ethyl)amino]-2-oxoethoxy}phenyl)ethyl]thio}benzoic acid

Methyl 2-{[2-(4-{2-[(4-chlorobenzyl)(ethyl)amino]-2-oxoethoxy}phenyl)-ethyl]thio }benzoate (0.085 g, 0.170 mmol) was dissolved in a mixture of acetonitrile/water (1/1, 4 ml) and lithium hydroxide (0.008 g, 0.341 mmol) was added. The reaction was performed in an single node microwave oven (5 min, 150 deg). The solvent was removed by evaporation and then HCl (2 ml, 1 M) was added. The water phase was extracted with two portions of EtOAc (20 ml). The combined organic phase was dried (MgSO₄) and the solvent was removed by evaporation and gave 0.073 g of 2-{[2-(4-{2-[(4-chlorobenzyl)(ethyl)amino]-2-oxoethoxy}phenyl)ethyl]thio}benzoic acid (yield 88.4%) as an oil which solidified on cooling and standing.
¹HNMR (rotamers, 400 MHz, CDCl₃): δ 1.09-1.20 (m, 3H), 2.91-2.98 (m, 2H), 3.11-3.17(m, 2H), 3.33-3.42 (m, 2H), 4.58-4.77 (m, 4H), 6.79, 6.92 (d, d, 2H), 7.11-7.49 (m, 9H), 8.10 (d, 1H).
¹³C NMR (rotamers, 100 MHz, CDCl₃): δ 12.23, 13.77, 33.70, 33.82, 41.09, 41.30, 47.42, 49.64, 67.37, 67.91, 114.69, 114.81, 123.97-135.58 (complex multiplet), 142.22, 156.45, 156.57, 168.20, 170.63.

The following two Examples were prepared in a similar manner:

### Example 4

2-[2-(4-{2-[(4-Chlorobenzyl)(ethyl)amino]-2-oxoethoxy}phenyl)ethoxy]benzoic acid.

### Example 5

2-[2-(4-{2-[Ethyl(4-trifluoromethylbenzyl)amino]-2-oxoethoxy}phenyl)ethoxy]benzoic acid.

### Example 6

a) Acetic acid (1.321g, 22.000 mmol) was dissolved in DMF (10 ml), 1-[4-(trifluoromethyl)phenyl]methanamine (3.468 g, 19.800 mmol) was added and the mixture was cooled to 0° C. *N*-[(1*H*-1,2,3-benzotriazol-1-yloxy)(dimethylamino)methylene]-*N-*methylmethanaminium tetrafluoroborate (7.770 g, 24.200 mmol) and *N*-ethyl-*N*,*N-*diisopropylamine (5.971 g, 46.200 mmol) was added. The solution was stirred for two hours at room temperature. EtOAc (20 ml) was added and the organic phase was washed with Na₂CO3 (3 X 20 ml, aq) and HCl (0.5 M, 2 X, 10 ml). The organic layer was dried (MgSO4) and the solvent was removed by evaporation. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions was pooled and the acetonitrile was removed by evaporation. EtOAc (10 ml) was added and the organic phase was washed with two portions of brine and dried (MgSO4) and the solvent was removed by evaporation and gave 3.085 g of *N*-[4-(trifluoromethyl)benzyl]acetamide (yield 64.6%).
   ¹HNMR (500 MHz, CDCl₃): δ 2.0 (s, 3H), 4.42 (d, 2H), 6.58 (bs, 1H), 7.35 (d, 2H), 7.55 (d, 2H)
b) *N*-[4-(trifluoromethyl)benzyl]acetamide (3.085 g, 14.204 mmol) was dissolved in THF (100 ml) and was cooled to zero degrees under argon atmosphere. (Methyltbio)methane compound with borane (1:1) (2.698 g, 35.511 mmol) was added and the mixture was refluxed over night at RT. HCl (15 ml, 10%) was gently added and was stirred overnight. The solvent was removed by evaporation. Diethyl ether (20 ml) was added and the product was extracted to the water phase by K₂CO₃ (3 X 15 ml), the water phase was acidified by HCl (10 ml, 10%) and the product was extracted to the organic phase by EtOAc (3 X 15 ml). The organic phase was dried (MgSO₄) and the solvent was removed by evaporation to give 0.809 g of *N*-[4-(trifluoromethyl)benzyl]ethanamine (yield 28%).
   ¹HNMR (500 MHz, CDCl₃): δ 1.05 (t, 3H), 1.3 (s, 1H), 2.62 (q, 2H), 3.78 (s, 2H), 7.38 (d, 2H), 7.3 (d, 2H)
c) *Tert*-butyl (4-{2-[(methylsulfonyl)oxy]ethyl}phenoxy)acetate (5.454 g, 17.347 mmol) was dissolved in acetonitrile (100 ml), methyl 2-mercaptobenzoate (3.502 g, 20.816 mmol) and dipotassium carbonate (4.795 g, 34.694 mmol) was added. The solution was stirred for 10 hours at 60 °C. EtOAc (40 ml) was added and the organic phase was washed with two portions of Brine (2 X 40 ml, aq). The organic layer was dried (MgSO₄) and the solvent was removed by evaporation to give 6.931 g crude of methyl 2-({2-[4-(2-*tert*-butoxy-2-oxoethoxy)phenyl]ethyl}thio)benzoate. The crude was used in the next step without further purification.
d) Methyl 2-({2-[4-(2-*tert*-butoxy-2-oxoethoxy)phenyl]ethyl}thio)benzoate (4.630 g, 11.502 mmol) was take up in DCM (50 ml) and treated with trifluoroacetic acid (44.40 g, 389.405 mmol) at r.t for 4h. The mixture was evaporated and azeotroped with toluene. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions was pooled and the acetonitrile was removed by evaporation. EtOAc (10 ml) was added and the organic phase was washed with two potions of brine and dried (MgSO4). The solvent was removed by evaporation to give 3.825 g of [4-(2-{[2-(methoxycarbonyl)phenyl]thio}ethyl)phenoxy]-acetic acid (yield for two steps 63.9% overall).
   ¹HNMR (500 MHz, CDCl₃): δ 2.82 (t, 2H), 3.15 (t, 2H), 3.82 (s, 3H), 4.35 (s, 2H), 6.78 (d, 2H), 7.18 (d, 2H), 7.23 (t, 1H), 7.51 (d, 1H), 7.55 (t, 1H), 7.85 (d, 1H).
e) [4-(2-{[2-(methoxycarbonyl)phenyl]thio}ethyl)phenoxy]acetic acid (0.200 g, 0.577 mmol) was dissolved in DMF (10 ml), *N*-[4-(trifluoromethyl)benzyl]ethanamine (0.129 g, 0.635 mmol) was added and the mixture was cooled to 0° C. *N*-[(1*H*-1,2,3-benzotriazol-1-yloxy)(dimethylamino)methylene]-*N*-methylmethanaminium tetrafluoroborate (0.204 g, 0.635 mmol) and *N*-ethyl-*N,N*-diisopropylamine (0.157 g, 1.212 mmol) was added. The solution was stirred overnight at room temperature. Water (100 ml) was added and the water phase was extracted with diethyl ether (3 X 20ml). The organic phase was washed with Na₂CO₃ (3 X 20 ml, aq) and HCl (0.5 M, 2 X, 10 ml). The organic layer was dried (MgSO4) and the solvent was removed by evaporation. The crude was purified by flash chromatography (started with isocratic heptane/EtOAc 30/70 and then the EtOAc concentration was increased to 100%, (silica gel 60 0.004-0.063 mm). The product containing fractions were pooled and the solvent was removed by evaporation to give 0.085 g of methyl 2-({2-[4-(2-{ethyl[4-(trifluoromethyl)benzyl]amino}-2-oxoethoxy)phenyl]ethyl}thio)benzoate (yield 27.7%).
   ¹HNMR (Rotamers, 500 MHz, CDCl₃): δ 1.1-1.23 (bm, 3H), 2.95 (q, 2H), 3.15 (q, 2H), 3.42 (m, 2H), 3.9 (s, 3H), 4.7-4.82 (bm, 4H), 6.75-6.95 (m, 2H), 7.1-7.5 (m, 9H), 7.97 (d, 1H).
f) Methyl 2-({2-[4-(2-{ethyl[4-(trifluoromethyl)benzyl]amino}-2-oxoethoxy)phenyl]-ethyl}thio)benzoate (0.085 g, 0.160 mmol) was dissolved in a mixture of acetonitrile/water (1/1, 4 ml), then lithium hydroxide (0.008 g, 0.320 mmol) was added. The reaction was performed in an single node microwave oven (5 min, 150 deg). Work-up by removing the solvent by evaporation and addition of HCl (2 ml, 1 M). The water phase was extracted with two portions of EtOAc (20 ml), the organic phase was dried (MgSO₄) and the solvent was removed by evaporation and gave 0.07773 g of 2-({2-[4-(2-{ethyl[4-(trifluoromethyl)benzyl]amino)-2-oxoethoxy)phenyl]ethyl}thio)benzoic acid (yield 93.0%).
   ¹HNMR (Rotamers, 400 MHz, CDCl₃): δ 1.02-1.25 (bm, 3H), 2.95 (q, 2H), 3.15 (q, 2H), 3.38 (m,2H), 4.60-4.82 (bm, 4H), 6.75-6.95 (m,2H), 7.1-7.5 (m, 9H), 7.97 (d, 1H).

### Example 7

Methyl 2-{2-[4-(2-{butyl[2-fluoro-4-(trifluoromethyl)benzyl]amino}-2-oxoethoxy)phenyl]ethoxy}benzoate (0.230 g, 0.410 mmol) was dissolved in a mixture of THF/ water (1/1, 4 ml). Lithium hydroxide (0.015 g, 0.617 mmol) was added. The reaction was performed in an single node microwave oven (14 min, 150 deg). Work-up by ° removing the solvent by evaporation and addition of HCl (2 ml, 1 M). The water-phase was extracted with two portions of EtOAc (20 ml). The organic phase was dried (MgSO4) and the solvent was removed by evaporation to give 0.212 g of 2-{2-[4-(2-{butyl[2-fluoro-4-(trifluoromethyl)benzyl]amino}-2-oxoethoxy)phenyl]ethoxy}benzoic acid (yield 94.5%).
¹HNMR (Rotamers, 500 MHz, CDCl₃): δ 0.82-1.0 (bm, 3H), 1.2-1.4 (bm, 2H), 1.65-1.7 (bm, 2H), 3.13 (m, 2H), 3.32 (m, 2H), 4.4 (m, 2H), 4.63-4.8 (M, 4H), 6.7-7.6 (bm, 10H), 8.1 (d, 1H).

### Example 8

a) (4-{2-[2-(Methoxycarbonyl)phenoxy]ethyl}phenoxy)acetic acid (0.150 g, 0.454 mmol) was dissolved in DMF (10 ml), *N*-(2,4-difluorobenzyl)-*N*-propylanline (0.084 g, 0.454 mmol) was added and the mixture was cooled to 0° C. *N*-[(1*H*-1,2,3-benzotriazol-1-yloxy)(dimethylamino)methylene]-*N*-methylmethanaminium tetrafluoroborate (0.160g, 0.499 mmol) and *N*-ethyl-*N,N*-diisopropylamine (0.123 g, 0.954 mmol) was added. The solution was stirred for two hours at room temperature. EtOAc (20 ml) was added and the organic phase was washed with Na₂CO₃ (3 X 20 ml, aq) and HCl (0.5 M, 2 X, 10 ml). The organic layer was dried (MgSO4) and the solvent was removed by evaporation to give 0.220 g of methyl 2-[2-(4-{2-[(2,4-difluorobenzyl)(propyl)amino]-2-oxoethoxy}phenyl)-ethoxy]benzoate (yield 97.4%).
   ¹HNMR (Rotamers, 500 MHz, CDCl₃): δ 0.8-1.0 (bm, 3H), 1.45-1.7 (bm, 2H) 3.1 (m, 2H), 3.28 (bm, 2H), 3.9 (s, 3H), 4.2 (q, 2H), 4.6-4.75 (M, 4H), 6.7-7.0 (bm, 6H), 7.1-7.3 (bm, 3H), 7.4 (m, 1H), 7.78 (d, 1H).
b) Methyl 2-[2-(4-{2-[(2,4-difluorobenzyl)(propyl)amino]-2-oxoethoxy}phenyl)-ethoxy]benzoate (0.22 g, 0.442 mmol) was dissolved in a mixture of THF/ water (1/1, 4 ml). Lithium hydroxide (0.021 g, 0.884 mmol) was added. The reaction was performed in an single node microwave oven (14 min, 150 deg). Work-up by removing the solvent by evaporation and addition of HCl (2 ml, 1 M). The waterphase was extracted with two portions of EtOAc (20 ml), the organic phase was dried (MgSO4) and the solvent was removed by evaporation to give 0.180 g of2-[2-(4-{2-[(2,4-difluorobenzyl)(propyl)-amino]-2-oxoethoxy}phenyl)ethoxy]benzoic acid (yield 84.2%).
   ¹HNMR (Rotamers, 500 MHz, CDCl₃): δ 0.8-1.0 (bm, 3H), 1.45-1.7 (bm, 2H), 3.14 (m, 2H), 3.28 (bm, 2H), 4.4 (q, 2H), 4.62 (s, 2H), 4.75 (s, 2H), 6.7-7.35 (bm, 9H), 7.52 (t, 1H), 8.12 (d, 1H).

### Example 9

a) (4-{2-[2-(methoxycarbonyl)phenoxy]ethyl}phenoxy)acetic acid (0.150 g, 0.454 mmol) was dissolved in DMF (10 ml), *N*-benzyl-*N*-ethylamine (0.061 g, 0.454 mmol) was added and the mixture was cooled to 0° C. *N*-[(1*H*-1,2,3-benzotriazol-1-yloxy)-(dimetliylamino)methylene]-*N*-methylmethanaminium tetrafluoroborate (0.160 g, 0.499 mmol) and *N*-ethyl-*N*,*N*-diisopropylamine (0.123 g, 0.954 mmol) was added. The solution was stirred for two hours at room temperature. EtOAc (20 ml) was added and the organic phase was washed with Na₂CO3 (3 X 20 ml, aq) and HCl (0.5 M, 2 X, 10 ml). The organic layer was dried (MgSO4) and the solvent was removed by evaporation to give 0.138 g of methyl 2-[2-(4-{2-[benzyl(ethyl)amino]-2-oxoethoxy}phenyl)ethoxy]benzoate (yield 67.9%).
   ¹HNMR (Rotamers, 500 MHz, CDCl₃): δ 1.07-1.22 (bm, 3H), 3.1 (m, 2H), 3.20 (bm, 2H), 3.9 (s, 3H), 4.2 (q, 2H), 4.6-4.8 (M, 4H), 6.8-7.02 (bm, 4H), 6.18-7.5 (bm, 8H), 7.78 (d, 1H).
b) Methyl 2-[2-(4-{2-[benzyl(ethyl)amino]-2-oxoethoxy}phenyl)ethoxy]benzoate (0.138 g, 0.308 mmol) was dissolved in a mixture of THF/ water (1/1, 4 ml). Lithium hydroxide (0.015 g, 0.617 mmol) was added. The reaction was performed in an single node microwave oven (14 min, 150 deg). Workup by removing the solvent by evaporation and addition of HCl (2 ml, 1 M). The waterphase was extracted with two portions of EtOAc (20 ml), the organic phase was dried (MgSO4) and the solvent was removed by evaporation to give 0.146 g of 2-[2-(4-{2-[benzyl(ethyl)amino]-2-oxoethoxy}phenyl)ethoxy]benzoic acid.
   ¹HNMR (Rotamers, 500 MHz, CDCl₃): δ 1.02-1.22 (bm, 3H), 3.1 (m, 2H), 3.25-3.5 (bm, 2H), 4.2 (q, 2H), 4.55-4.8 (M, 4H), 6.8-7.4 (bm, 11H), 7.5 (m, 1H), 8.1 (d, 1H).

### Example 10

a) *Tert*-butyl (4-{2-[(methylsulfonyl)oxy]ethyl}phenoxy)acetate (5.454 g, 17.347 mmol) was dissolved in acetonitrile (100 ml), methyl 2-mercaptobenzoate (3.502 g, 20.816 mmol) and dipotassium carbonate (4.795 g, 34.694 mmol) was added. The solution was stirred for 10 hours at 60°C. EtOAc (40 ml) was added and the organic phase was washed with two portions of brine (2 X 40 ml, aq). The organic layer was dried (MgSO₄) and the solvent was removed by evaporation to give 6.931 g crude of methyl 2-({2-[4-(2-*tert*-butoxy-2-oxoethoxy)phenyl]ethyl}thio)benzoate. The crude was used in the next step without further purification.
b) Methyl 2-({2-[4-(2-*tert*-butoxy-2-oxoethoxy)phenyl]ethyl}thio)benzoate (4.630 g, 11.502 mmol) was take up in DCM (50 ml) and treated with trifluoroacetic acid (44.40 g, 389.405 mmol) at r.t for 4h. The mixture was evaporated and azeotroped with toluene. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. EtOAc (10 ml) was added and the organic phase was washed with two portions of brine and dried (MgSO₄). The solvent was removed by evaporation to give 3.825 g of [4-(2-{[2-(methoxycarbonyl)phenyl]thio}ethyl)-phenoxy]acetic acid (yield for two steps 63.9% overall).
   ¹HNMR (500 MHz, CDCl₃): δ 2.82 (t, 2H), 3.15 (t, 2H), 3.82 (s, 3H), 4.35 (s, 2H), 6.78 (d, 2H), 7.18 (d, 2H), 7.23 (t, 1H), 7.51 (d, 1H), 7.55 (t, 1H), 7.85 (d, 1H).
c) [4-(2-{[2-(methoxycarbonyl)phenyl]thio}ethyl)phenoxy]acetic acid (0.200 g, 0.577 mmol) was dissolved in DMF (10 ml), *N*-benzyl-*N*-ethylamine (0.086 g, 0.635 mmol) was added and the mixture was cooled to 0° C. *N*-[(1*H*-1,2,3-benzotriazol-1-yloxy)(dimethylamino)methylene]-*N*-methylmethanaminium tetrafluoroborate (0.204 g, 0.635 mmol) and *N*-ethyl-*N,N*-diisopropylamine (0.157 g, 1.212 mmol) was added. The solution was stirred over night at room temperature. Water (100 ml) was added and the water phase was extracted with diethyl ether (3 X 20ml). The organic phase was washed with Na₂CO₃ (3 X 20 ml, aq) and HCl (0.5 M, 2 X, 10 ml). The organic layer was dried (MgSO4) and the solvent was removed by evaporation. The crude was purified by flash chromatography (started with isocratic heptane/EtOAc 30/70 and then the EtOAc concentration was increased to 100%, (silica gel 60 0.004-0.063 mm). The product containing fractions were pooled and the solvent was removed by evaporation to give 0.137 g of methyl 2-{[2-(4-{2-[benzyl(ethyl)amino]-2-oxoethoxy}phenyl)-ethyl]thio }benzoate (yield 51.2%).
d) Methyl2-{[2-(4-{2-[benzyl(ethyl)amino]-2-oxoethoxy}phenyl)ethyl]thio}benzoate (0.137 g, 0.296 mmol) was dissolved in a mixture of acetonitrile/ water (1/1, 4 ml), lithium hydroxide (0.014 g, 0.591 mmol) was added. The reaction was performed in an single node microwave oven (5 min, 150 deg). Work-up by removing the solvent by evaporation and addition of HCl (2 ml, 1 M). The waterphase was extracted with two portions of EtOAc (20 ml), the organic phase was dried (MgSO₄) and the solvent was removed by evaporation and gave 0.111g of 2-{[2-(4-{2-[benzyl(ethyl)amino]-2-oxoethoxy}phenyl)ethyl]-thio}benzoic acid (yield 83.5%).
   ¹HNMR (Rotamers, 400 MHz, CDCl₃): δ 1.02-1.30 (bm, 3H), 2.95 (q, 2H), 3.15 (q, 2H), 3.40 (m, 2H), 4.58 (s, 2H), 4.63-4.92 (bm, 4H), 6.85-7.0 (bm, 2H), 7.0-7.53 (m, 10H), 7.97 (d, 1H).

### Example 11

a) *N*-(4-*tert*-butylbenzyl)-*N*-ethylamine (0.143 g, 0.746 mmol) was dissolved in dry acetonitrile under N₂ and *N*-ethyl-*N*,*N*-diisopropylamine (0.371 g, 2.867 mmol) was added. The mixture was stirred for 30 min and methyl 2-{2-[4-(2-chloro-2-oxoethoxy)phenyl]ethoxy}benzoate (0.200 g, 0.573 mmol) was added. The solution was stirred overnight at room temperature. The crude was purified by flash cromatography (started with isocratic heptane/EtOAc 50/50 and then the EtOAc concentration was increased to 100%, (silica gel 60 0.004-0.063 mm). The product containing fractions were pooled and the EtOAc was removed by evaporation to give 0.229 g of methyl 2-[2-(4-{2-[(4-*tert*-butylbenzyl)(ethyl)amino]-2-oxoethoxy}phenyl)ethoxy]benzoate (yield 79.3%).
   ¹HNMR (Rotamers, 500 MHz, CDCl₃): δ 1.07-1.23 (bm, 3H), 2.23 (m, 9H), 3.08 (m, 2H), 3.30-3.5 (bm, 2H), 3.87 (s, 3H), 4.18 (m, 2H), 4.58 (d, 2H), 4.63-4.8 (m, 2H) 6.77-7.43 (m, 11H), 7.78 (d, 1H).
b) Methyl 2-[2-(4-{2-[(4-*tert*-butylbenzyl)(ethyl)amino]-2-oxoethoxy}phenyl)-ethoxy]benzoate (0.2290 g, 0.455 mmol)was dissolved in a mixture ofTHF (freshly distilled)/ water (2/1, 3 ml), lithium-hydroxide (0.218 g, 0.909 mmol) was added. The reaction was performed in a single node microwave oven (5 min, 150 deg). THF was removed by evaporation. Water was added (10ml) and the basic water phase was washed with diethyl ether (2 X 10 ml). Addition of HCl (2 ml, 1 M, pH 1). The water phase was extracted with two portions of DCM (20 ml), the organic phase was dried (MgSO4) and the solvent was removed by evaporation to give 0.163 g of 2-[2-(4-{2-[(4-*tert-*butylbenzyl)(ethyl)amino]-2-oxoethoxy}phenyl)ethoxy]benzoic acid (yield 73.2%). ¹HNMR (Rotamers, 500 MHz, CDCl₃): δ 1.07-1.21 (bm, 3H), 2.28 (m, 9H), 3.12 (m, 2H), 3.28-3.5 (bm, 2H), 4.4 (m, 2H), 4.58 (d, 2H), 4.63-4.78 (m, 2H) 6.80-7.55 (m, 11H), 8.1 (d, 1H).

### Example 12

a) Acetic acid (1.321g, 22.000 mmol) was dissolved in DMF (10 ml), 1-(4-fluorophenyl)methanamine (2.478 g, 19.800 mmol) was added and the mixture was cooled to 0° C. *N*-[(1*H*-1,2,3-beuzotriazol-1-yloxy)(dimethylamino)methylene]-*N-*methylmethanaminium tetrafluoroborate (7.770 g, 24.200 mmol) and *N*-ethyl-*N*,*N* diisopropylamine (5.971 g, 46.200 mmol) was added. The solution was stirred for two hours at room temperature. EtOAc (20 ml) was added and the organic phase was washed with Na₂CO3 (3 X 20 ml, aq) and HCl (0.5 M, 2 X, 10 ml). The organic layer was dried (MgSO4) and the solvent was removed by evaporation. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mmX 250 mm, flow 40 ml/min). The product containing fractions was pooled and the acetonitrile was removed by evaporation. EtOAc (10 ml) was added and the organic phase was washed with two portions of brine and dried (MgSO4) and the solvent was removed by evaporation and gave 1.344 g of *N*-(4-fluorobenzyl)acetamide (yield 36.5%).
   ¹HNMR (500 MHz, CDCl₃): δ 1.98 (s, 3H), 4.35 (d, 2H), 6.25 (bs, 1H), 6.95-7.25 (bm, 4H)
b) *N*-(4-Fluorobenzyl)acetamide (1.344 g, 8.039 mmol) was dissolved in THF (100 ml) and was cooled to zero degrees under argon atmosphere. (Methylthio)methane compound with borane (1:1) (1.527 g, 20.098 mmol) was added and the mixture was refluxed overnight at RT. HCl (15 ml, 10%) was gently added and was stirred overnight. The solvent was removed by evaporation. Diethyl ether (20 ml) was added and the product was extracted to the water phase by K₂CO₃ (3 X 15 ml). The water phase was acidified by HCl (10 ml, 10%) and the product was extracted to the organic phase by EtOAc (3 X 15 ml). The organic phase was dried (MgSO₄) and the solvent was removed by evaporation to give 0.309 g of *N*-(4-fluorobenzyl)ethanamine (yield 25.1 %).
   ¹HNMR (400 MHz, CDCl₃): δ 1.05 (t, 3H), 1.1 (s, 1H), 2.58 (q, 2H), 3.64 (s, 2H), 6.9 (t, 2H), 7.2 (t, 2H).
c) *N*-(4-Fluorobenzyl)ethanamine (0.114 g, 0.745 mmol) was dissolved in dry acetonitrile under N₂ and *N*-ethyl-*N*,*N*-diisopropylamine (0.371 g, 2.867 mmol) was added. The mixture was stirred for 30 min and methyl 2-{2-[4-(2-chloro-2-oxoethoxy)phenyl]-ethoxy}benzoate (0.200 g, 0.573 mmol) was added. The solution was stirred overnight at room temperature. The crude was purified by flash chromatography (started with isocratic heptane/EtOAc 50/50 and then the EtOAc concentration was increased to 100%, (silica gel 60 0.004-0.063 mm). The product containing fractions were pooled and the EtOAc was removed by evaporation to give 0.223 g of methyl 2-[2-(4-{2-[ethyl(4-fluorobenzyl)amino]-2-oxoethoxy}phenyl)ethoxy]benzoate (yield 83.5%).
   ¹HNMR (Rotamers, 500 MHz, CDCl₃): δ 1.07-1.23 (bm, 3H), 3.08 (m, 2H), 3.30-3.45 (bm, 2H), 3.87 (s, 3H), 4.18 (m, 2H), 4.58 (s, 2H), 4.63-4.8 (m, 2H) 6.77-7.45 (m, 11H), 7.78 (d, 1H).
d) Methyl 2-[2-(4-{2-[ethyl(4-fluorobenzyl)amino]-2-oxoethoxy}phenyl)ethoxy]-benzoate (0.223 g, 0.479 mmol) was dissolved in a mixture of THF (freshly distilled)/water (2/1,3 ml), lithium hydroxide (0.229 g, 0.958 mmol) was added. The reaction was performed in a single node microwave oven (5 min, 150 deg). THF was removed by evaporation. Water was added (10ml) and the basic water phase was washed with diethyl ether (2 X 10 ml). Addition of HCl (2 ml, 1 M, pH 1). The water phase was extracted with two portions of DCM (20 ml), the organic phase was dried (MgSO4) and the solvent was removed by evaporation to give 0.196 g of 2-[2-(4-{2-[ethyl(4-fluorobenzyl)amino]-2-oxoethoxy}phenyl)ethoxy]benzoic acid (yield 90.6%).
   ¹HNMR (Rotamers, 500 MHz, CDCl₃): δ 1.03-1.21 (bm, 3H), 3.1 (m, 2H), 3.23-3.4 (bm, 2H), 4.38 (m, 2H), 4.55 (s, 2H), 4.63-4.78 (m, 2H), 6.75-7.22 (m, 10H), 7.47 (t, 1H), 8.07 (d, 1H).

### Example 13

a) *Tert*-butyl (4-{2-[(methylsulfonyl)oxy]ethyl}phenoxy)acetate (5.454 g, 17.347 mmol) was dissolved in acetonitrile (100 ml), methyl 2-mercaptobenzoate (3.502 g, 20.816 mmol) and dipotassium carbonate (4.795 g, 34.694 mmol) was added. The solution was stirred for 10 hours at 60°C. EtOAc (40 ml) was added and the organic phase was washed with two portions of Brine (2 X 40 ml, aq). The organic layer was dried (MgSO₄) and the solvent was removed by evaporation to give 6.931 g crude of methyl 2-({2-[4-(2-*tert*-butoxy-2-oxoethoxy)phenyl]ethyl}thio)benzoate. The crude was used in the next step without further purification.
b) Methyl 2-({2-[4-(2-*tert*-butoxy-2-oxoethoxy)phenyl]ethyl}thio)benzoate (4.630 g, 11.502 mmol) was take up in DCM (50 ml) and treated with trifluoroacetic acid (44.40 g, 389.405 mmol) at r.t for 4h. The mixture was evaporated and azeotroped with toluene. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. EtOAc (10 ml) was added and the organic phase was washed with two portions of brine and dried (MgSO4). The solvent was removed by evaporation to give 3.825 g of [4-(2-{[2-(methoxycarbonyl)phenyl]thio}ethyl)phenoxy]-acetic acid (yield for two steps 63.9% overall).
   ¹HNMR (500 MHz, CDCl₃): δ 2.82 (t, 2H), 3.15 (t, 2H), 3.82 (s, 3H), 4.35 (s, 2H), 6.78 (d, 2H), 7.18 (d, 2H), 7.23 (t, 1H), 7.51 (d, 1H), 7.55 (t, 1H), 7.85 (d, 1H).
c) [4-(2-{[2-(Methoxycarbonyl)phenyl]thio}ethyl)pbenoxy]acetic acid (0.250 g, 0.722 mmol) was dissolved in DCM (10 ml), *N*-(2-fluorobenzyl)ethanamine (0.105 g, 0.686 mmol) was added. *N*-[(1*H*-1,2,3-benzotriazol-1-yloxy)(dimethylamino)methylene]-*N-*methylmethanaminium tetrafluoroborate (0.255 g, 0.0.794 mmol) and *N*-ethyl-*N*,*N-*diisopropylamine (0.187 g, 1.443 mmol) were added. The solution was stirred for 2 hours at room temperature. The crude was purified by flash chromatography (started with isocratic DCM 100% and then the MeOH concentration was increased from 0.5% to 20%, (silica gel 60 0.004-0.063 mm). The product containing fractions were pooled and the EtOAc was removed by evaporation. The substance needed to be purified once more and it was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. The ammonium acetate was removed by freeze drying the product overnight to give 0.1 g of methyl 2-{[2-(4-{2-[ethyl(2-fluorobenzyl)amino]-2-oxoethoxy}phenyl)ethyl]thio}benzoate (yield 29.1%).
   ¹HNMR (Rotamers, 500 MHz, CDCl₃): δ 1.03-1.23 (bm, 3H), 2.95 (q, 2H), 3.15 (q, 2H), 3.40 (m, 2H), 3.9 (s, 3H), 4.6-4.8 (bm, 4H), 6.75-6.95 (m, 2H), 6.97-7.5 (m, 9H), 7.97 (d, 1H).
d) Methyl 2-{[2-(4-{2-[ethyl(2-fluorobenzyl)amino]-2-oxoethoxy}phenyl)ethyl]thio}-benzoate was dissolved in a mixture of THF (freshly distilled)/ water (2/1, 3 ml), Lithiuim hydroxide (0.015 g, 0.629 mmol) was added. The reaction was performed in a single node microwave oven (5 min, 150 deg). THF was removed by evaporation. Water was added (10ml) and the basic water phase was washed with diethyl ether (2 X 10 ml). Addition of HCl (2 ml, 1 M, pH 1). The water phase was extracted with two portions of DCM (20 ml), the organic phase was dried (MgSO4) and the solvent was removed by evaporation to give 0.095 g of 2-{[2-(4-{2-[ethyl(2-fluorobenzyl)amino]-2-oxoethoxy}phenyl)ethyl]-thio}benzoic acid (yield 96.9%)
   ¹HNMR (Rotamers, 400 MHz, CDCl₃): δ 1.02-1.25 (bm, 3H), 2.95 (q, 2H), 3.15 (q, 2H), 3.42 (m, 2H), 4.60-4.80 (bm, 4H), 6.75-6.95 (m, 2H), 6.95-7.5 (m, 9H), 8.1 (d, 1H).

### Example 14

a) *Tert*-butyl (4-{2-[(methylsulfonyl)oxy]ethyl}phenoxy)acetate (5.454 g, 17.347 mmol) was dissolved in acetonitrile (100 ml), methyl 2-mercaptobenzoate (3.502 g, 20.816 mmol) and dipotassium carbonate (4.795 g, 34.694 mmol) was added. The solution was stirred for 10 hours at 60°C. EtOAc (40 ml) was added and the organic phase was washed with two portions of brine (2 X 40 ml, aq). The organic layer was dried (MgSO₄) and the solvent was removed by evaporation to give 6.931 g crude of methyl 2-({2-[4-(2-*tert*-butoxy-2-oxoethoxy)phenyl]ethyl}thio)benzoate. The crude was used in the next step without further purification.
b) Methyl 2-({2-[4-(2-*tert*-butoxy-2-oxoethoxy)phenyl]ethyl}thio)benzoate (4.630 g, 11.502 mmol) was take up in DCM (50 ml) and treated with trifluoroacetic acid (44.40 g, 389.405 mmol) at r.t for 4h. The mixture was evaporated and azeotroped with toluene. The crude was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. EtOAc (10 ml) was added and the organic phase was washed with two portions of brine and dried (MgSO4). The solvent was removed by evaporation to give 3.825 g of [4-(2-{[2-(methoxycarbonyl)phenyl]thio}ethyl)-phenoxy]acetic acid (yield for two steps 63.9% overall).
   ¹HNMR (500 MHz, CDCl₃): δ 2.82 (t, 2H), 3.15 (t, 2H), 3.82 (s, 3H), 4.35 (s, 2H), 6.78 (d, 2H), 7.18 (d, 2H), 7.23 (t, 1H), 7.51 (d, 1H), 7.55 (t, 1H), 7.85 (d, 1H).
c) [4-(2-{[2-(methoxycarbonyl)phenyl]thio}ethyl)phenoxy]acetic acid (0.250 g, 0.722 mmol) was dissolved in DCM (10 ml) and *N*-(2-chlorobenzyl)-*N*-ethylamine (0.116 g, 0.686 mmol) was added. *N*-[(1*H*-1,2,3-Benzotriazol-1-yloxy)(dimethylamino)methylene]-*N*-methylmethanaminium tetrafluoroborate (0.255 g, 0.0.794 mmol) and *N*-ethyl-*N,N-*diisopropylamine (0.187 g, 1.443 mmol) were added. The solution was stirred for 2 hours at room temperature. The crude was purified by flash chromatography (started with isocratic DCM 100% and then the MeOH concentration was increased from 0.5% to 20%, (silica gel 60 0.004-0.063 mm). The product containing fractions were pooled and the EtOAc was removed by evaporation. The substance needed to be purified once more and it was purified by preparative HPLC (started with isocratic acetonitrile/buffer 60/40 and then the acetonitrile concentration was increased to 100%, the buffer was a mixture of acetonitrile/water 10/90 and ammonium acetate (0.1 M, column KR-100-7-C8, 50 mm X 250 mm, flow 40 ml/min). The product containing fractions were pooled and the acetonitrile was removed by evaporation. The ammonium acetate was removed by freeze drying the product overnight to give 0.111 g of methyl 2-{[2-(4-{2-[(2-chlorobenzyl)(ethyl)amino]-2-oxoethoxy}phenyl)ethyl]thio}benzoate (yield 30.9%).
   ¹HNMR (Rotamers, 500 MHz, CDCl₃): δ 1.10-1.25 (bm, 3H), 2.95 (m, 2H), 3.15 (m, 2H), 3.40 (m, 2H), 3.9 (s, 3H), 4.6-4.8 (bm, 4H), 6.75-6.95 (m, 2H), 7.02-7.5 (m, 9H), 7.95 (d, 1H).
d) Methyl 2-{[2-(4-{2-[(2-chlorobemyl)(ethyl)amino]-2-oxoethoxy}phenyl)ethyl]thio}-benzoate was dissolved in a mixture of THF (freshly distilled)/ water (2/1, 3 ml), Lithiuim hydroxide (0.015 g, 0.629 mmol) was added. The reaction was performed in a single node microwave oven (5 min, 150 deg). THF was removed by evaporation. Water was added (10ml) and the basic water phase was washed with diethyl ether (2 X 10 ml). Addition of HCl (2 ml, 1 M, pH 1). The water phase was extracted with two portions of DCM (20 ml). The organic phase was dried (MgSO4) and the solvent was removed by evaporation to give 0.103 g of 2-{[2-(4-{2-[(2-chlorobenzyl)(ethyl)amino]-2-oxoethoxy}phenyl)ethyl]thio}-benzoic acid (yield 95.5%)
   ¹HNMR (Rotamers, 400 MHz, CDCl₃): δ 1.07-1.25 (bm, 3H), 2.95 (m, 2H), 3.15 (m, 2H), 3.42 (m, 2H), 4.62-4.85 (bm, 4H), 6.75-6.95 (m, 2H), 7.02-7.5 (m, 9H), 8.1 (d, 1H).

### Biological activity

### Formulations

Compounds were dissolved in DMSO to obtain 16 mM stock solutions. Before assays, stock solutions were further diluted in DMSO and culture media.

### GENERAL CHEMICALS AND REAGENTS

Luciferase assay reagent was purchased from Packard, USA. Restriction Enzymes were from Boehringer and Vent polymerase from New England Biolabs.

### CELL LINES AND CELL CULTURE CONDITIONS

U2-OS, (Osteogenic sarcoma, Human) was purchased from ATCC, USA. Cells were expanded and refrozen in batches from passage number six. Cells were cultured in Dulbecco's modified Eagle medium (DMEM) with 25 mM glucose, 2 mM glutamine or 4 mM L-alanyl-L-glutamine,10% fetal calf serum, at 5% CO₂. Phosphate buffered saline (PBS) without addition of calcium or magnesium was used. All cell culture reagents were from Gibco (USA) and 96-well cell culture plates were purchased from Wallach

### PLASMID CONSTRUCTS FOR HETEROLOGOUS EXPRESSION

Standard recombinant DNA techniques were carried out as described by Ausubel (7). The Luciferase reporter vector, pGL5UAS (clone consists of five copies of the GAL4 DNA binding sequence, 5'-CGACGGAGTACTGTCCTCCGAGCT-3', cloned into the SacI/XhoI sites of pGL3-Promoter (Promega). The SacI/XhoI fragment carrying the UAS sites was constructed using annealed overlapping oligonucleotides.

Expression vectors used are based upon pSG5 (Stratagene). All vectors contain an EcoRI/NheI fragment encoding the DNA binding domain of GAL4 (encoding amino acid positions 1-145 of database accession number P04386) followed by an in-frame fusion to a fragment encoding the nuclear localisation sequence from T antigen of Polyoma Virus. The nuclear localisation sequence was constructed using annealed overlapping oligonucleotides creating NheI/KpnI sticky ends
(5'-CTAGCGCTCCTAGAAGAAACGCAAGGTTGGTAC-3'). The ligand binding domains from human and mouse PPARα and human and mouse PPARγ were PCR amplified as KpnI/BamHI fragments and cloned in frame to the GAL4 DNA binding domain and the nuclear localisation sequence. The sequence of all plasmid constructs used were confirmed by sequencing.
The following expression vectors were used for transient transfections:

| vector | encoded PPAR subtype | sequence reference¹ |
|---|---|---|
| pSGGALhPPa | human PPARα | S74349, nt 625-1530 |
| pSGGALmPPa | murine PPARα | X57638, nt 668-1573 |
| pSGGALhPPg | human PPARγ | U63415, nt 613-1518 |
| pSGGALmPPg | murine PPARγ | U09138, nt 652-1577 |

| | | |
|---|---|---|
| ¹ refers to nucleotide positions of data base entry used to express the ligand binding domain. | | |

### TRANSIENT TRANSFECTIONS

Frozen stocks of cells from passage number six were thawed and expanded to passage number eight before transfections. Confluent cells were trypsinised washed and pelleted by centrifugation at 270xg for 2 minutes. The cell pellet was resuspended in cold PBS to a cell concentration of about 18 x 10⁶ cells/ml. After addition of DNA, the cell suspension was incubated on ice for approximately 5 minutes before electroporation at 230 V, 960 µF in Biorad's Gene Pulser^{™} in 0.5 ml batches. A total of 50 µg DNA was added to each batch of 0.5 ml cells, including 2.5 µg expression vector, 25 µg reporter vector and 22.5 µg unspecific DNA (pBluescript, Stratagene).

After electroporation, cells were diluted to a concentration of 320'000 cells/ml in DMEM without phenol red, and approximately 25'000 cells/well were seeded in 96-well plates. In order to allow cells to recover, seeded plates were incubated at 37°C for 3-4 hours before addition of test compounds. In assays for PPARα, the cell medium was supplemented with resin-charcoal stripped fetal calf serum (FCS) in order to avoid background activation by fatty acid components of the FCS. The resin-charcoal stripped FCS was produced as follows; for 500 ml of heat-inactivated FCS, 10 g charcoal and 25 g Bio-Rad Analytical Grade Anion Exchange Resin 200-400 mesh were added, and the solution was kept on a magnetic stirrer at room temperature over night. The following day, the FCS was centrifuged and the stripping procedure was repeated for 4-6 hours. After the second treatment, the FCS was centrifuged and filter sterilised in order to remove remnants of charcoal and resin.

### ASSAY PROCEDURE

Stock solutions of compounds in DMSO were diluted in appropriate concentration ranges in master plates. From master plates, compounds were diluted in culture media to obtain test compound solutions for final doses.

After adjustment of the amount of cell medium to 75 µl in each well, 50 µl test compound solution was added. Transiently transfected cells were exposed to compounds for about 24 hours before the luciferase detection assay was performed. For luciferase assays, 100 µl of assay reagent was added manually to each well and plates were left for approximately 20 minutes in order to allow lysis of the cells. After lysis, luciferase activity was measured in a 1420 Multiwell counter, Victor, from Wallach.

### Reference compounds

The TZD pioglitazone was used as reference substance for activation of both human and murine PPARγ. 5,8,11,14-Eicosatetrayonic acid (ETYA) was used as reference substance for human PPARα.

### Calculations and analysis

For calculation of EC₅₀ values, a concentration-effect curve was established. Values used were derived from the average of two or three independent measurements (after subtraction of the background average value) and were expressed as the percentage of the maximal activation obtained by the reference compound. Values were plotted against the logarithm of the test compound concentration. EC₅₀ values were estimated by linear intercalation between the data points and calculating the concentration required to achieve 50% of the maximal activation obtained by the reference compound.

The compounds of formula I have an EC₅₀ of less than 50µmol/l for PPARα and preferred compounds have an EC₅₀ of less than 5µmol/l. For example the EC₅₀s of some of the Examples for human PPAR alpha are:
Example 3 0.499µmol/l; and
Example 5 0.048 µmol/l.

## Claims

1. A compound of formula I 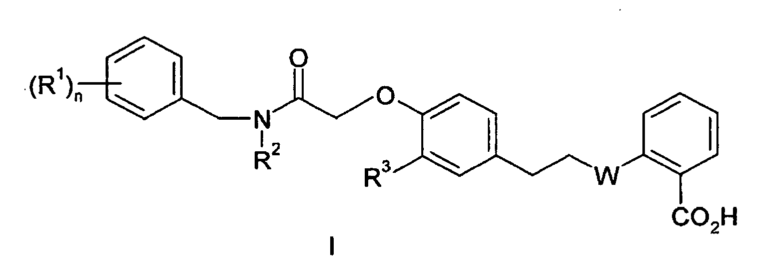 wherein n is 0, 1 or 2;
R¹ represents halo, a C₁₋₄alkyl group which is optionally substituted by one or more fluoro, a C₁₋₄alkoxy group which is optionally substituted by one or more fluoro and wherein when n is 2 the substituents R¹ may be the same or different;
R² represents an unbranched C₂₋₇alkyl group;
R³ represents H or OCH₃; and
W represents O or S;
and pharmaceutically acceptable salts thereof.

2. A compound according to claim 1 wherein W is O.

3. A compound according to claim 1 wherein W is S.

4. A compound according to claim 1 selected from:
2-[2-(4-{2-[ethyl(2-fluorobenzyl)amino]-2-oxoethoxy}phenyl)ethoxy]benzoic acid;
2-[2-(4-{2-[(2,4-difluorobenzyl)(heptyl)amino]-2-oxoethoxy}-3-methoxyphenyl)-ethoxy]benzoic acid;
2-[2-(4-{2-[(4-chlorobenzyl)(ethyl)amino]-2-oxoethoxy}phenyl)ethylthio]benzoic acid;
2-[2-(4-{2-[(4-chlorobenzyl)(ethyl)amino]-2-oxoethoxy}phenyl)ethoxy]benzoic acid;
2-[2-(4-{2-[ethyl(4-trifluoromethylbenzyl)amino]-2-oxoethoxy}phenyl)ethylthio]benzoic acid;
2-[2-(4-{2-[ethyl(4-trifluoromethylbenzyl)amino]-2-oxoethoxy}phenyl)ethoxy]benzoic acid;
2-{2-[4-(2-{butyl[2-fluoro-4-(trifluoromethyl)benzyl]amino}-2-oxoethoxy)phenyl]ethoxy}benzoic acid;
2-[2-(4-{2-[(2,4-difluorobenzyl)(propyl)amino]-2-oxoethoxy}phenyl)ethoxy]benzoic acid;
2-[2-(4-{2-[benzyl(ethyl)amino]-2-oxoethoxy}phenyl)ethoxy]benzoic acid;
2-{[2-(4-{2-[benzyl(ethyl)amino]-2-oxoethoxy}phenyl)ethyl]thio}benzoic acid;
2-[2-(4-{2-[(4-*tert*-butylbenzyl)(ethyl)amino]-2-oxoethoxy}phenyl)ethoxy]benzoic acid;
2-[2-(4-{2-[ethyl(4-fluorobenzyl)amino]-2-oxoethoxy}phenyl)ethoxy]benzoic acid;
2- {[2-(4-{2-[ethyl(2-fluorobenzyl)amino]-2-oxoethoxy}phenyl)ethyl]thio}benzoic acid; or
2-{[2-(4-{2-[(2-chlorobenzyl)(ethyl)amino]-2-oxoethoxy}phenyl)ethyl]thio}benzoic acid
and pharmaceutically acceptable salts thereof.

5. A pharmaceutical formulation comprising a compound according to any preceding claim in admixture with pharmaceutically acceptable adjuvants, diluents and/or carriers.

6. The use of a compound according to any one of claims 1 to 4 in the manufacture of a medicament for the treatment of insulin resistance.

7. A process to prepare a compound of formula I which comprises reacting a compound of formula II 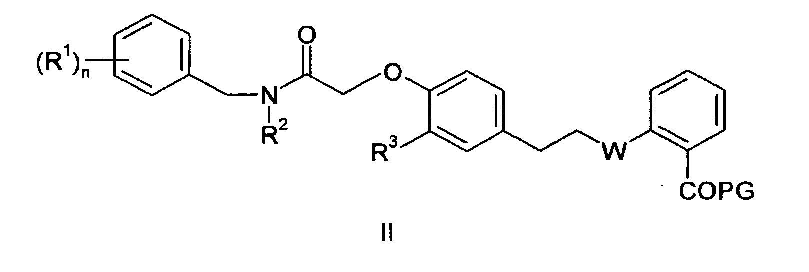 in which R¹, R², R³, W and n are as previously defined and PG represents a protecting group for a carboxylic hydroxy group with a de-protecting agent.

8. A compound of formula II as described in claim 7.

## Patentansprüche

1. Verbindung der Formel I 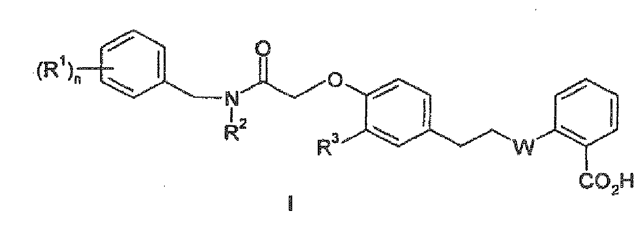 worin n für 0, 1 oder 2 steht;
R¹ für Halogen, eine C₁₋₄-Alkylgruppe, die gegebenenfalls durch ein oder mehrere Fluoratome substituiert ist, eine C₁₋₄-Alkoxygruppe, die gegebenenfalls durch ein oder mehrere Fluoratome substituiert ist, steht und dann, wenn n für 2 steht, die Substituenten R¹ gleich oder verschieden sein können;
R² für eine unverzweigte C₂₋₇-Alkylgruppe steht;
R³ für H oder OCH₃ steht und
W für O oder S steht;
und pharmazeutisch annehmbare Salze davon.

2. Verbindung nach Anspruch 1, worin W für O steht.

3. Verbindung nach Anspruch 1, worin W für S steht.

4. Verbindung nach Anspruch 1, ausgewählt unter:
2-[2-(4-{2-[Ethyl(2-fluorbenzyl)amino]-2-oxo-ethoxy}phenyl)ethoxy]benzoesäure;
2-[2-(4-{2-[(2,4-Difluorbenzyl)(heptyl)amino]-2-oxo-ethoxy}-3-methoxyphenyl)ethoxy]benzoesäure;
2-[2-(4-{2-[(4-Chlorbenzyl)(ethyl)amino]-2-oxo-ethoxyl}phenyl)ethylthio]benzoesäure;
2-[2-(4-{2-[(4-Chlorbenzyl)(ethyl)amino]-2-oxo-ethoxy}phenyl)ethoxy]benzoesäure;
2-[2-(4-{2-[Ethyl(4-trifluormethylbenzyl)amino]-2-oxo-ethoxy}phenyl)ethylthio]benzoesäure;
2-[2-(4-{2-[Ethyl(4-trifluormethylbenzyl)amino]-2-oxo-ethoxy}phenyl)ethoxy]benzoesäure;
2-{2-[4-(2-{Butyl[2-fluor-4-(trifluormethyl)benzyl]-amino}-2-oxoethoxy)phenyl]ethoxy}benzoesäure;
2-[2-(4-{2-[(2,4-Difluorbenzyl)(propyl)amino]-2-oxo-ethoxy}phenyl)ethoxy]benzoesäure;
2-[2-(4-{2-[Benzyl(ethyl)amino]-2-oxoethoxy}-phenyl)ethoxy]benzoesäure;
2-{[2-(4-{2-[Benzyl(ethyl)amino]-2-oxoethoxy}-phenyl)ethyl]thio}benzoesäure;
2-[2-(4-{2-[(4-tert-Butylbenzyl)(ethyl)amino]-2-oxo-ethoxy}phenyl)ethoxy]benzoesäure;
2-[2-(4-{2-[Ethyl(4-fluorbenzyl)amino]-2-oxo-ethoxy}phenyl)ethoxy]benzoesäure;
2-{[2-(4-{2-[Ethyl(2-fluorbenzyl)amino]-2-oxo-ethoxy}phenyl)ethoxy]thio}benzoesäure oder 2-{[2-(4-{2-[(2-Chlorbenzyl)(ethyl)amino]-2-oxo-ethoxy}phenyl)ethoxy]thio}benzoesäure;
und pharmazeutisch annehmbare Salze davon.

5. Pharmazeutische Formulierung, enthaltend eine Verbindung nach einem der vorhergehenden Ansprüche in Abmischung mit pharmazeutisch annehmbaren Hilfsstoffen, Verdünnungsmitteln und/oder Trägern.

6. Verwendung einer Verbindung nach einem der Ansprüche 1 bis 4 bei der Herstellung eines Arzneimittels zur Behandlung von Insulinresistenz.

7. Verfahren zur Herstellung einer Verbindung der Formel I, bei dem man eine Verbindung der Formel II 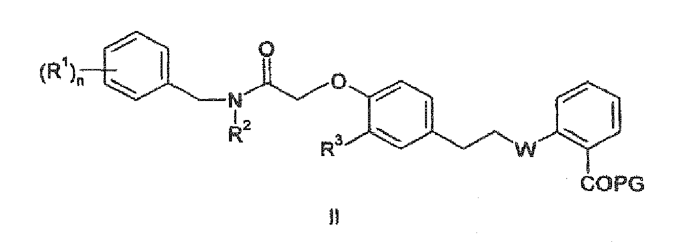 worin R¹, R², R³, W und n die oben angegebene Bedeutung besitzen und PG für eine Schutzgruppe für eine Carbonsäure-Hydroxylgruppe steht, mit einem Entschützungsmittel umsetzt.

8. Verbindung der Formel II gemäß Anspruch 7.

## Revendications

1. Composé de formule I 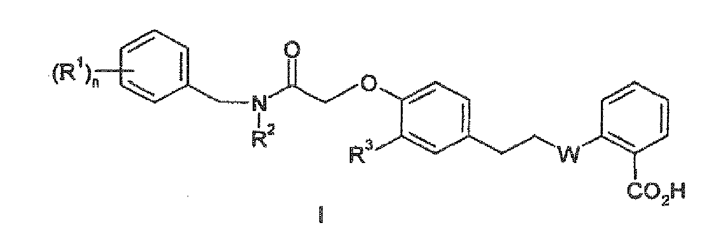 dans laquelle n vaut 0, 1 ou 2 ;
R¹ représente un groupe halogéno, un groupe alkyle en C₁₋₄ qui est éventuellement substitué par un ou plusieurs groupes fluoro, un groupe alcoxy en C₁₋₄ qui est éventuellement substitué par un ou plusieurs groupes fluoro, et dans laquelle lorsque n vaut 2, les substituants R¹ peuvent être identiques ou différents ;
R² représente un groupe alkyle en C₂₋₇ non ramifié ;
R³ représente un atome d'hydrogène ou un groupe OCH₃ ; et
W représente un atome d'oxygène ou un atome de soufre ; et leurs sels pharmaceutiquement acceptables.

2. Composé selon la revendication 1, dans lequel W est un atome d'oxygène.

3. Composé selon la revendication 1, dans lequel W est un atome de soufre.

4. Composé selon la revendication 1 choisi parmi :
l'acide 2-[2-(4-{2-[éthyl(2-fluorobenzyl)amino]-2-oxoéthoxy}phényl)éthoxy]benzoïque ;
l'acide 2- [2- (4-{2- [(2,4-difluorobenzyl) (heptyl) amino]-2-oxoéthoxy}-3-méthoxyphényl)éthoxy]benzoïque ;
l'acide 2-[2-(4-{2-[(4-chlorobenzyl)(éthyl)amino]-2-oxoéthoxy}phényl)éthylthio]benzoïque ;
l'acide 2-[2-(4-{2-[(4-chlorobenzyl)(éthyl)amino]-2-oxoéthoxy}phényl)éthoxy]benzoïque ;
l'acide 2-[2-(4-{2-[éthyl(4-trifluorométhylbenzyl)-amino]-2-oxoéthoxy}phényl)éthylthio]benzoïque ;
l'acide 2-[2-(4-{2-[éthyl(4-trifluorométhylbenzyl)-amino]-2-oxoéthoxy}phényl)éthoxy]benzoïque ;
l'acide 2-{2-[4-(2-{butyl[2-fluoro-4-(trifluorométhyl)benzyl]amino}-2-oxoéthoxy)phényl]éthoxy}benzoïque ;
l'acide 2- [2- (4-{2- [(2,4-difluorobenzyl) (propyl) amino]-2-oxoéthoxy}phényl)éthoxy]benzoïque ;
l'acide 2-[2-(4-{2-[benzyl(éthyl)amino]-2-oxoéthoxy}phényl)éthoxy]benzoïque ;
l'acide 2-{[2-(4-{2-[benzyl(éthyl)amino]-2-oxoéthoxy}phényl)éthyl]thio}benzoïque ;
l'acide 2-[2-(4-{2-[(4-tert-butylbenzyl)(éthyl)amino]-2-oxoéthoxy}phényl)éthoxy]benzoïque ;
l'acide 2-[2-(4-{2-[éthyl(4-fluorobenzyl)amino]-2-oxoéthoxy}phényl)éthoxy]benzoïque ;
l'acide 2-{[2-(4-{2-[éthyl(2-fluorobenzyl)amino]-2-oxoéthoxy}phényl)éthyl]thio}benzoïque ; ou
l'acide 2-{[2-(4-{2-[(2-chlorobenzyl)(éthyl)amino]-2-oxoéthoxy}phényl)éthyl]thio}benzoïque ;
et leurs sels pharmaceutiquement acceptables.

5. Formulation pharmaceutique comprenant un composé selon l'une quelconque des revendications précédentes en mélange avec des adjuvants, des diluants et/ou des supports pharmaceutiquement acceptables.

6. Utilisation d'un composé selon l'une quelconque des revendications 1 à 4 pour la fabrication d'un médicament destiné au traitement de la résistance à l'insuline.

7. Procédé de préparation d'un composé de formule I, comprenant la réaction d'un composé de formule II 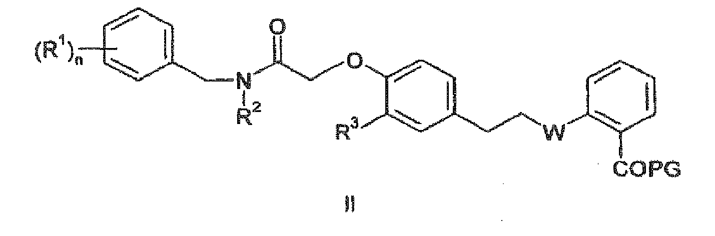 dans laquelle R¹, R², R³, W et n sont tels que définis précédemment et PG représente un groupe protecteur pour un groupe hydroxycarboxylique, avec un agent de déprotection.

8. Composé de formule II tel que décrit dans la revendication 7.
